(19) Europäisches Patentamt / European Patent Office / Office européen des brevets

(11) EP 1 910 330 B1

(12) # EUROPÄISCHE PATENTSCHRIFT

(45) Veröffentlichungstag und Bekanntmachung des Hinweises auf die Patenterteilung:
**23.07.2014 Patentblatt 2014/30**

(21) Anmeldenummer: **06754657.2**

(22) Anmeldetag: **01.07.2006**

(51) Int Cl.:
***C07D 401/12*** $^{(2006.01)}$ ***C07D 409/12*** $^{(2006.01)}$
***C07D 417/12*** $^{(2006.01)}$ ***A61K 31/40*** $^{(2006.01)}$
***A61K 31/38*** $^{(2006.01)}$

(86) Internationale Anmeldenummer:
**PCT/EP2006/006434**

(87) Internationale Veröffentlichungsnummer:
**WO 2007/009578 (25.01.2007 Gazette 2007/04)**

(54) **HETEROCYCLYLAMID-SUBSTITUIERTE THIAZOLE, PYRROLE UND THIOPHENE**
HETEROCYCLYLAMIDE-SUBSTITUTED THIAZOLES, PYRROLES AND THIOPHENES
THIAZOLES, PYRROLES ET THIOPHENES SUBSTITUÉS PAR UN HÉTÉROCYCLYLAMIDE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **15.07.2005 DE 102005033103**

(43) Veröffentlichungstag der Anmeldung:
**16.04.2008 Patentblatt 2008/16**

(73) Patentinhaber: **AiCuris GmbH & Co. KG**
**42117 Wuppertal (DE)**

(72) Erfinder:
- **ZIMMERMANN, Holger**
  **42111 Wuppertal (DE)**
- **BRÜCKNER, David**
  **45128 Essen (DE)**
- **HENNINGER, Kerstin**
  **42115 Wuppertal (DE)**
- **HENDRIX, Martin**
  **51519 Odenthal (DE)**
- **RADTKE, Martin**
  **40699 Erkrath (DE)**

(74) Vertreter: **Kilger, Ute et al**
**Boehmert & Boehmert**
**Pettenkoferstrasse 20-22**
**80336 München (DE)**

(56) Entgegenhaltungen:
**WO-A-2004/002481 WO-A-2004/052852**

- **BURAK K ET AL: "SYNTHESIS OF ISOTHIAZOLE DERIVATIVES WITH POTENTIAL BIOLOGICAL ACTIVITY" PHARMAZIE, DIE, GOVI VERLAG, ESCHBORN, DE, Bd. 47, Nr. 7, 1992, Seiten 492-495, XP000651576 ISSN: 0031-7144**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 910 330 B1

## Beschreibung

Die Erfindung betrifft Heterocyclylamid-substituierte Thiazole, Pyrrole und Thiophene und Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere zur Verwendung als antivirale Mittel, insbesondere gegen Cytomegalieviren.

WO 99/23091 beschreibt aromatische heterocyclische Verbindungen als antiinflammatorische Mittel, die unter anderem auch zur Behandlung von viralen Infektionen geeignet sein können und WO 04/052852 beschreibt 3-Pyrrolyl-Harnstoffderivate als antivirale Mittel, die einen Carbocyclus als Substituenten am Harnstoff tragen.

Auf dem Markt sind zwar strukturell andersartige antiviral wirkende Mittel vorhanden, aber die gegenwärtig verfügbaren Therapien mit Ganciclovir, Valganciclovir, Foscamet und Cidofovir sind mit schweren Nebenwirkungen verbunden, z.B. Nephrotoxizität, Neutropenie oder Thrombozytopenie. Zudem kann es regelmäßig zu einer Resistenzentwicklung kommen. Neue Mittel für eine wirksame Therapie sind daher wünschenswert.

Eine Aufgabe der vorliegenden Erfindung ist es daher, neue Verbindungen mit gleicher oder verbesserter antiviraler Wirkung zur Behandlung von viralen Infektionskrankheiten bei Menschen und Tieren zur Verfügung zu stellen.

Überraschenderweise wurde gefunden, dass die in der vorliegenden Erfmdung beschriebenen substituierten Heterozyklen antiviral hochwirksam sind.

Gegenstand der vorliegenden Erfindung sind Verbindungen der Formel

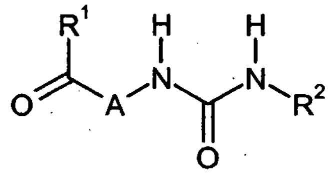

(I),

in welcher

$R^1$ für eine Gruppe der Formel

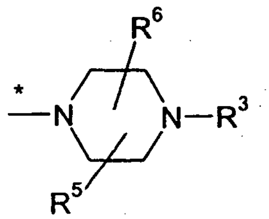

oder

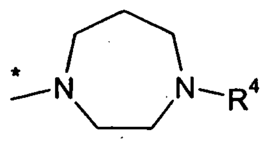

steht,
wobei

* für die Anknüpfstelle an die Carbonyl-Gruppe steht,

$R^3$ für Phenyl oder 5- oder 6-gliedriges Heteroaryl steht, worin Phenyl und Heteroaryl substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Difluormethyl, Trifluormethoxy, Difluormethoxy, Monofluormethoxy, Trifluormethylthio, $C_1$-$C_6$-Alkyl, $C_1$-$C_6$-Alkoxy, Hydroxycarbonyl, $C_1$-$C_6$-Alkoxycarbonyl, Amino, $C_1$-$C_6$-Alkylamino, Aminocarbonyl und $C_1$-$C_6$-Alkylaminocarbonyl,

$R^4$ für Phenyl oder 5- oder 6-gliedriges Heteroaryl steht, worin Phenyl und Heteroaryl substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Difluormethyl, Trifluormethoxy, Difluormethoxy, Monofluormethoxy, Trifluormethylthio, $C_1$-$C_6$-Alkyl, $C_1$-$C_6$-Alkoxy, Hydroxycarbonyl, $C_1$-$C_6$-Alkoxycarbonyl, Amino, $C_1$-$C_6$-Alkylamino, Aminocarbonyl und $C_1$-$C_6$-Alkylaminocarbonyl, und

$R^5$ und $R^6$ unabhängig voneinander für Wasserstoff, Methyl oder Ethyl stehen,

$R^2$ für Phenyl steht,
wobei Phenyl substituiert sein kann mit 1 bis 3 Substituenten, wobei die Substituenten unabhängig voneinander ausgewählt werden aus der Gruppe, bestehend aus Halogen, Hydroxy, Trifluormethyl, Difluormethyl, Trifluormethoxy, Difluormethoxy, Monofluormethoxy, Trifluormethylthio, $C_1$-$C_6$-Alkyl und $C_1$-$C_6$-Alkoxy,

A für eine Gruppe der Formel

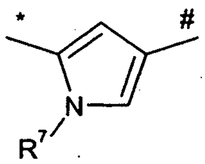
,
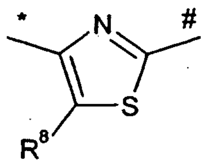
,
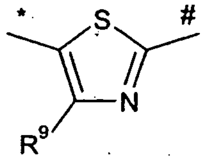
oder
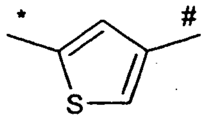

steht,
wobei

* für die Anknüpfstelle an die Carbonyl-Gruppe steht,

\# für die Anknüpfstelle an das Stickstoffatom des Harnstoffs steht,

$R^7$ für $C_1$-$C_6$-Alkyl steht,
wobei Alkyl substituiert sein kann mit einem Substituenten, wobei der Substituent ausgewählt wird aus der Gruppe, bestehend aus $C_3$-$C_6$-Cycloalkyl, $C_6$-$C_{10}$-Aryl und 5- oder 6-gliedriges Heteroaryl,
worin Cycloalkyl, Aryl und Heteroaryl substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Difluormethyl, Trifluormethoxy, Difluormethoxy, Monofluormethoxy, Trifluormethylthio, $C_1$-$C_6$-Alkyl, $C_1$-$C_6$-Alkoxy, Hydroxycarbonyl, $C_1$-$C_6$-Alkoxycarbonyl, Amino, $C_1$-$C_6$-Alkylamino, Aminocarbonyl und $C_1$-$C_6$-Alkylaminocarbonyl,
und

$R^8$ und $R^9$ unabhängig voneinander für Wasserstoff, Halogen oder $C_1$-$C_6$-Alkyl stehen,
wobei Alkyl substituiert sein kann mit einem Substituenten, wobei der Substituent ausgewählt wird aus der Gruppe, bestehend aus $C_3$-$C_6$-Cycloalkyl, $C_6$-$C_{10}$-Aryl und 5- oder 6-gliedriges Heteroaryl,
worin Cycloalkyl, Aryl und Heteroaryl substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Difluormethyl, Trifluormethoxy, Difluormethoxy, Monofluormethoxy, Trifluormethylthio, $C_1$-$C_6$-Alkyl, $C_1$-$C_6$-Alkoxy, Hydroxycarbonyl, $C_1$-$C_6$-Alkoxycarbonyl, Amino, $C_1$-$C_6$-Alkylamino, Aminocarbonyl und $C_1$-$C_6$-Alkylaminocarbonyl,

und ihre Salze, ihre Solvate und die Solvate ihrer Salze.

Erfindungsgemäße Verbindungen sind die Verbindungen der Formel (I) und deren Salze, Solvate und Solvate der Salze; die von Formel (I) umfassten Verbindungen der nachfolgend genannten Formeln und deren Salze, Solvate und Solvate der Salze sowie die von Formel (I) umfassen, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Außerdem umfasst die vorliegende Erfindung auch Prodrugs der erfindungsgemäßen Verbindungen. Der Begriff "Prodrugs" umfasst Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:

Alkyl per se und "Alk" und "Alkyl" in Alkoxy, Alkylamino, Alkoxycarbonyl und Alkylaminocarbonyl stehen für einen linearen oder verzweigten Alkylrest mit in der Regel 1 bis 6 ("$C_1$-$C_6$-Alkyl"), vorzugsweise 1 bis 4, besonders bevorzugt 1 bis 3 Kohlenstoffatomen, beispielhaft und vorzugsweise für Methyl, Ethyl, n-Propyl, Isopropyl, tert.-Butyl, n-Pentyl und n-Hexyl.

Alkoxy steht beispielhaft und vorzugsweise für Methoxy, Ethoxy, n-Propoxy, Isopropoxy, tert-Butoxy, n-Pentoxy und n-Hexoxy.

Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, tert.-Butylamino, n-Pentylamino, n-Hexylamino, *N,N*-Dimethylamino, *N,N*-Diethylamino, N-Ethyl-*N*-methylamino, *N*-Methyl-*N*-n-propylamino, *N*-Isopropyl-N-n-propylamino, *N*-t-Butyl-*N*-methylamino, *N*-Ethyl-N-n-pentylamino und *N*-n-Hexyl-*N*-methylamino. $C_1$-$C_3$-Alkylamino steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent.

Alkoxycarbonyl steht beispielhaft und vorzugsweise für Methoxycarbonyl, Ethoxycarbonyl, n-Propoxycarbonyl, Isopropoxycarbonyl, tert.-Butoxycarbonyl, n-Pentoxycarbonyl und n-Hexoxycarbonyl.

Alkylaminocarbonyl steht für einen Alkylaminocarbonylrest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, Isopropylaminocarbonyl, tert.-Butylaminocarbonyl, n-Pentylaminocarbonyl, n-Hexylaminocarbonyl, *N,N*-Dimethylaminocarbonyl, *N,N*-Diethylaminocarbonyl, *N*-Ethyl-*N*-methylaminocarbonyl, *N*-Methyl-*N*-n-propylaminocarbonyl, *N*-Isopropyl-*N*-n-propylaminocarbonyl, *N*-tert.-Butyl-*N*-methylaminocarbonyl, *N*-Ethyl-*N*-n-pentylamino-carbonyl und *N*-n-Hexyl-*N*-methylaminocarbonyl. $C_1$-$C_3$-Alkylaminnocarbonyl steht beispielsweise für einen Monoalkylaminocarbonylrest mit 1 bis 3 Kohlenstoffatomen oder für einen Dialkylaminocarbonylrest mit jeweils 1 bis 3 Kohlenstoffatomen pro Alkylsubstituent.

Aryl steht für einen mono- oder bicyclischen aromatischen, carbocyclischen Rest mit in der Regel 6 bis 10 Kohlenstoffatomen; beispielhaft und vorzugsweise für Phenyl und Naphthyl.

5- oder 6-gliedriges Heteroaryl steht im Rahmen der Erfindung im allgemeinen für einen aromatischen, monocyclischen Rest mit 5 oder 6 Ringatomen und bis zu 4 Heteroatomen aus der Reihe S, O und/oder N. Der Heteroarylrest kann über ein Kohlenstoff- oder Heteroatom gebunden sein. Beispielsweise und vorzugsweise seien genannt: Thienyl, Furyl, Pyrrolyl, Thiazolyl, Oxazolyl, Pyrazolyl, Imidazolyl, Pyridyl, Pyrimidyl, Pyridazinyl.

Cycloalkyl steht für eine Cycloalkylgruppe mit in der Regel 3 bis 6 Kohlenstoffatomen, beispielhaft und vorzugsweise für Cyclopropyl, Cyclobutyl, Cyclopentyl, Cyclohexyl und Cycloheptyl.

Halogen steht für Fluor, Chlor, Brom und Jod.

Bevorzugt im Rahmen der vorliegenden Erfindung sind Verbindungen der Formel (I),

in welcher

$R^1$ für eine Gruppe der Formel

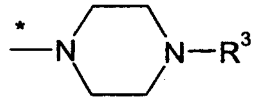

steht,
wobei

* für die Anknüpfstelle an die Carbonyl-Gruppe steht,

$R^3$ für Phenyl oder 5- oder 6-gliedriges Heteroaryl steht,
worin Phenyl und Heteroaryl substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Difluormethyl, Trifluormethoxy, Difluormethoxy, Monofluormethoxy, Trifluormethylthio, $C_1$-$C_6$-Alkyl, $C_1$-$C_6$-Alkoxy, Hydroxycarbonyl, $C_1$-$C_6$-Alkoxycarbonyl, Amino, $C_1$-$C_6$-Alkylamino, Aminocarbonyl und $C_1$-$C_6$-Alkylaminocarbonyl,

$R^2$ für Phenyl steht,
wobei Phenyl substituiert sein kann mit 1 bis 3 Substituenten, wobei die Substituenten unabhängig voneinander ausgewählt werden aus der Gruppe, bestehend aus Halogen, Hydroxy, Trifluormethyl, Difluormethyl, Trifluormethoxy, Difluormethoxy, Monofluormethoxy, Trifluormethylthio, $C_1$-$C_6$-Alkyl und $C_1$-$C_6$-Alkoxy,

A für eine Gruppe der Formel

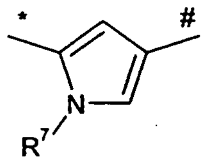
,
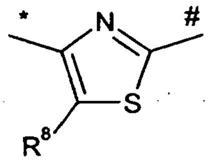
,

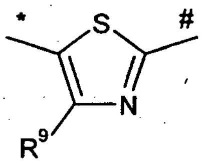
,
oder
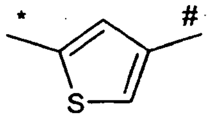

steht,
wobei

* für die Anknüpfstelle an die Carbonyl-Gruppe steht,

# für die Anknüpfstelle an das Stickstoffatom des Harnstoffs steht,

$R^7$ für $C_1$-$C_6$-Alkyl steht,
wobei Alkyl substituiert sein kann mit einem Substituenten, wobei der Substituent ausgewählt wird aus der Gruppe, bestehend aus $C_3$-$C_6$-Cycloalkyl, $C_6$-$C_{10}$-Aryl und 5- oder 6-gliedriges Heteroaryl,
worin Cycloalkyl, Aryl und Heteroaryl substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Difluormethyl, Trifluormethoxy, Difluormethoxy, Monofluormethoxy, Trifluormethylthio, $C_1$-$C_6$-Alkyl, $C_1$-$C_6$-Alkoxy, Hydroxycarbonyl, $C_1$-$C_6$-Alkoxycarbonyl, Amino, $C_1$-$C_6$-Alkylamino, Aminocarbonyl und $C_1$-$C_6$-Alkylaminocarbonyl,

und

$R^8$ und $R^9$ unabhängig voneinander für Wasserstoff, Halogen oder $C_1$-$C_6$-Alkyl stehen,

und ihre Salze, ihre Solvate und die Solvate ihrer Salze.

Bevorzugt im Rahmen der vorliegenden Erfindung sind auch Verbindungen der Formel (I), in welcher

$R^1$ für eine Gruppe der Formel

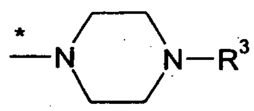

steht,
wobei

* für die Anknüpfstelle an die Carbonyl-Gruppe steht,

$R^3$ für Phenyl oder Pyridyl steht, worin Phenyl und Pyridyl substituiert sein können mit 1 bis 3 Substituenten, wobei die Substituenten unabhängig voneinander ausgewählt werden aus der Gruppe, bestehend aus Halogen, Nitro, Cyano, Trifluormethyl, Difluormethyl, Trifluormethoxy, Difluormethoxy, Monofluormethoxy, $C_1$-$C_4$-Alkyl und $C_1$-$C_4$-Alkoxy,

$R^2$ für Phenyl steht, wobei Phenyl substituiert sein kann mit 1 bis 3 Substituenten, wobei die Substituenten unabhängig voneinander ausgewählt werden aus der Gruppe, bestehend aus Fluor, Chlor, Trifluormethoxy, Difluormethoxy, Trifluormethylthio und Methyl,

A für eine Gruppe der Formel

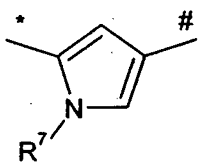 , 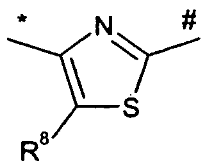 ,

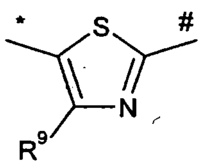 oder 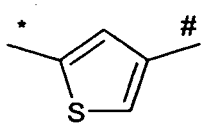

stehet,
wobei

* für die Anknüpfstelle an die Carbonyl-Gruppe steht,

\# für die Anknüpfstelle an das Stickstoffatom des Harnstoffs steht,

$R^7$ für Methyl, Ethyl oder n-Butyl steht, wobei Methyl, Ethyl und n-Butyl substituiert sein können mit einem Substituenten, wobei der Substituent ausgewählt wird aus der Gruppe, bestehend aus Cyclopropyl und Phenyl, worin Phenyl substituiert sein kann mit einem Substituenten Trifluormethyl,

und

$R^8$ und $R^9$ unabhängig voneinander für Wasserstoff, Brom, Chlor, Methyl oder Ethyl stehen,

und ihre Salze, ihre Solvate und die Solvate ihrer Salze.

Bevorzugt im Rahmen der vorliegenden Erfindung sind auch Verbindungen der Formel (I), in welcher $R^1$ für eine Gruppe der Formel

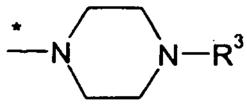

steht,
wobei

* für die Anknüpfstelle an die Carbonyl-Gruppe steht, und

$R^3$ für Phenyl oder Pyridyl steht,
worin Phenyl und Pyridyl substituiert sein können mit 1 bis 3 Substituenten, wobei die Substituenten unabhängig voneinander ausgewählt werden aus der Gruppe, bestehend aus Halogen, Nitro, Cyano, Trifluormethyl, Difluormethyl, Trifluormethoxy, Difluormethoxy, Monofluormethoxy, $C_1$-$C_4$-Alkyl und $C_1$-$C_4$-Alkoxy.

Bevorzugt im Rahmen der vorliegenden Erfindung sind auch Verbindungen der Formel (I), in welcher $R^2$ für Phenyl steht, wobei Phenyl substituiert sein kann mit 1 bis 3 Substituenten, wobei die Substituenten unabhängig voneinander ausgewählt werden aus der Gruppe, bestehend aus Fluor, Chlor, Trifluormethoxy, Difluormethoxy, Trifluormethylthio und Methyl.

Bevorzugt im Rahmen der vorliegenden Erfindung sind auch Verbindungen der Formel (I), in welcher A für eine Gruppe der Formel

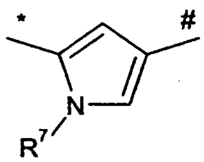 , 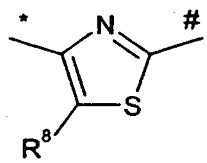 ,

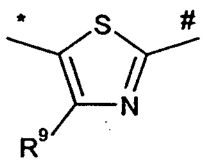 oder 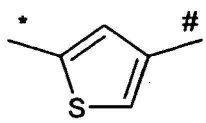

steht,
wobei

* für die Anknüpfstelle an die Carbonyl-Gruppe steht,
\# für die Anknüpfstelle an das Stickstoffatom des Harnstoffs steht,
$R^7$ für Methyl, Ethyl oder n-Butyl steht,
wobei Methyl, Ethyl und n-Butyl substituiert sein können mit einem Substituenten, wobei der Substituent ausgewählt wird aus der Gruppe, bestehend aus Cyclopropyl und Phenyl,
worin Phenyl substituiert sein kann mit einem Substituenten Trifluormethyl,
$R^8$ für Wasserstoff, Brom, Chlor oder Methyl steht,
und
$R^9$ für Wasserstoff steht.

Gegenstand der Erfindung ist weiterhin ein Verfahren zur Herstellung der Verbindungen der Formel (I), wobei

nach Verfahren [A] Verbindungen der Formel

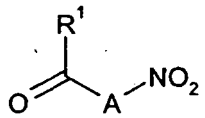

(II),

in welcher
$R^1$ die oben angegebene Bedeutung hat,
in der ersten Stufe mit einem Reduktionsmittel und in der zweiten Stufe in Gegenwart eines Kohlensäurederivates mit Verbindungen der Formel

$H_2N-R^2$ (III),

in welcher
$R^2$ die oben angegebene Bedeutung hat,
oder
nach Verfahren [B] Verbindungen der Formel (II) in der ersten Stufe mit einem Reduktionsmittel und in der zweiten Stufe mit Verbindungen der Formel

$OCN-R^2$ (IV),

in welcher

$R^2$ die oben angegebene Bedeutung hat,

oder
nach Verfahren [C] Verbindungen der Formel

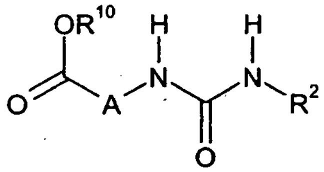

(V),

in welcher

$R^2$ die oben angegebene Bedeutung hat, und
$R^{10}$ für Methyl oder Ethyl steht,

in der ersten Stufe mit einer Base und in der zweiten Stufe mit Verbindungen der Formel

$R^1$-H (VI),

in welcher

$R^1$ die oben angegebene Bedeutung hat,

in Gegenwart von Dehydratisierungsreagenzien umgesetzt werden.
Die Verbindungen der Formeln (III), (IV) und (VI) sind bekannt oder lassen sich nach bekannten Verfahren aus den entsprechenden Edukten synthetisieren.

Für Verfahren [A] und [B] 1. Stufe gilt:

Die Umsetzung erfolgt im Allgemeinen in inerten Lösungsmitteln, bevorzugt in einem Temperaturbereich von 0°C bis zum Rückfluss der Lösungsmittel bei Normaldruck bis 3 bar.
Reduktionsmittel sind beispielsweise Palladium auf Aktivkohle und Wasserstoff, Ameisensäure/Triethylamin/Palladium auf Aktivkohle, Zink, Zink/Salzsäure, Eisen, Eisen/Salzsäure, Eisen-(II)sulfat/Salzsäure, Natriumsulfid, Natriumdisulfid Natriumdithionit, Ammoniumpolysulfid, Natriumborhydrid/Nickelchlorid, Zinndichlorid, Titantrichlorid oder Raney-Nickel und wässrige Hydrazin-Lösung, bevorzugt ist Raney-Nickel und wässrige Hydrazin-Lösung, Palladium auf Aktivkohle und Wasserstoff oder Ameisensäure/Triethylamin/Palladium auf Aktivkohle.

Inerte Lösungsmittel sind beispielsweise Ether wie Diethylether, Methyl-tert.-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Acetonitril oder Pyridin, im Falle von wassermischbaren Lösungsmitteln auch Gemische derselben mit Wasser, als Lösungsmittel ist bevorzugt Methanol, Ethanol, iso-Propanol oder im Falle von Raney-Nickel und wässrige Hydrazin-Lösung Tetrahydrofuran.

Für Verfahren [A] 2. Stufe gilt:

Die Umsetzung erfolgt im Allgemeinen in inerten Lösungsmitteln, bevorzugt in einem Temperaturbereich von Raumtemperatur bis 40°C bei Normaldruck.

Kohlensäurederivate sind beispielsweise N,N-Carbonyldiimidazol, Phosgen, Diphosgen, Triphosgen, Chlorameisensäurephenylester oder Chlorameisensäure-4-nitrophenylester, bevorzugt ist N,N-Carbonyldiimidazol.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Ethylacetat, Aceton, Dimethylformamid, Dimethylacetamid, 2-Butanon, Dimethylsulfoxid, Acetonitril oder Pyridin, im Falle von wassermischbaren Lösungsmitteln auch Gemische derselben mit Wasser, bevorzugt ist Dimethylsulfoxid.

Für Verfahren [B] 2. Stufe gilt:

Die Umsetzung erfolgt im Allgemeinen in inerten Lösungsmitteln, gegebenenfalls in Gegenwart einer Base, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Ethylacetat, Aceton, Dimethylformamid, Dimethylacetamid, 2-Butanon, Dimethylsulfoxid, Acetonitril oder Pyridin, bevorzugt sind Tetrahydrofuran oder Methylenchlorid.

Basen sind beispielsweise Alkalicarbonate wie Cäsiumcarbonat, Natrium- oder Kaliumcarbonat, oder Kalium-tert.-butylat, oder andere Basen wie Natriumhydrid, DBU, Triethylamin oder Diisopropylethylamin, bevorzugt Triethylamin.

Für Verfahren [C] 1. Stufe gilt:

Die Umsetzung erfolgt im Allgemeinen in inerten Lösungsmitteln, bevorzugt in einem Temperaturbereich von 0°C bis zum Rückfluss der Lösungsmittel bei Normaldruck.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, bevorzugt Natriumhydroxid.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Dimethylsulfoxid, Acetonitril oder Pyridin, oder Gemische von Lösungsmitteln mit Wasser, als Lösungsmittel ist bevorzugt ein Gemisch aus Ethanol und Wasser.

Für Verfahren [C] 2. Stufe gilt:

Die Umsetzung erfolgt im Allgemeinen in inerten Lösungsmitteln, gegebenenfalls in Gegenwart einer Base, bevorzugt in einem Temperaturbereich von -70°C bis 40°C bei Normaldruck.

Als Dehydratisierungsreagenzien eignen sich hierbei beispielsweise Carbodiimide wie z.B. N,N'-Diethyl-, N,N,'-Dipropyl-, N,N'-Diisopropyl-, N,N'-Dicyclohexylcarbodiimid, N-(3-Dimethylaminoisopropyl)-N'-ethylcarbodiimid-Hydrochlorid (EDC), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N'-tetra-methyluronium-hexafluorophosphat (HBTU), 2-(2-Oxo-1-(2H)-pyridyl)-1,1,3,3-tetramethyluroniumtetrafluoro-borat (TPTU) oder O-(7-Azabenzotiazol-1-yl)-N,N,N,N'-tetramethyl-uroniumhexafluorophosphat (HATU) oder 1-Hydroxybenztriazol (HOBt) oder Benzotriazol-1-yloxytris(dimethylamino)-phosphoniumhexafluorophosphat (BOP), oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4-Dimethylaminopyridin (DMAP) oder Diisopropylethylamin, oder DBU, DBN, Pyridin, bevorzugt ist Triethylamin.

Vorzugsweise wird die Kondensation mit TBTU und DMAP durchgeführt.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Ethylacetat, Aceton, Dimethylformamid, Dimethylacetamid, 2-butanon, Dimethylsulfoxid, Acetonitril oder Pyridin, im Falle von wassermischbaren Lösungsmitteln auch Gemische derselben mit Wasser, bevorzugt ist Dimethylformamid.

In einem alternativen Verfahren können die aus der ersten Stufe des Verfahrens [C] erhaltenen Carbonsäuren in der zweiten Stufe zunächst mit einem Chlorierungsreagenz wie beispielsweise Thionylchlorid zum Carbonsäurechlorid und anschließend mit Verbindungen der Formel (VI) in Gegenwart einer Base zu Verbindungen der Formel (I) umgesetzt werden.

Die Verbindungen der Formel (II) sind bekannt oder können hergestellt werden, indem Verbindungen der Formel

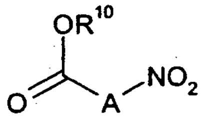 (VII),

in welcher

$R^{10}$ oben angegebene Bedeutung hat,

in der ersten Stufe mit einer Base und in der zweiten Stufe mit Verbindungen der Formel (VI), in Gegenwart von Dehydratisierungsreagenzien umgesetzt werden.

Die Umsetzung erfolgt wie in Verfahren [C] beschrieben.

Die Verbindungen der Formel (VII) sind bekannt oder können hergestellt werden, indem Verbindungen der Formel

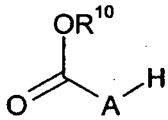 (VIII),

in welcher

$R^{10}$ die oben angegebene Bedeutung hat,

mit rauchender Salpetersäure, konzentrierter Salpetersäure, Nitriersäure oder anderen Mischungsverhältnissen von Schwefelsäure und Salpetersäure, gegebenenfalls in Acetanhydrid als Lösungsmittel, bevorzugt in einem Temperaturbereich von Raumtemperatur bis 60°C bei Normaldruck, umgesetzt werden.

Die Verbindungen der Formel (VIII) sind bekannt oder lassen sich nach bekannten Verfahren aus den entsprechenden Edukten synthetisieren.

Die Verbindungen der Formel (V) sind bekannt oder können hergestellt werden, indem Verbindungen der Formel (VII) in der ersten Stufe mit einem Reduktionsmittel und in der zweiten Stufe in Gegenwart eines Kohlensäurederivates mit Verbindungen der Formel (III) oder in der zweiten Stufe mit Verbindungen der Formel (IV) umgesetzt werden.

Die Umsetzung erfolgt wie in Verfahren [A] und [B] beschrieben.

Syntheseschema 1:

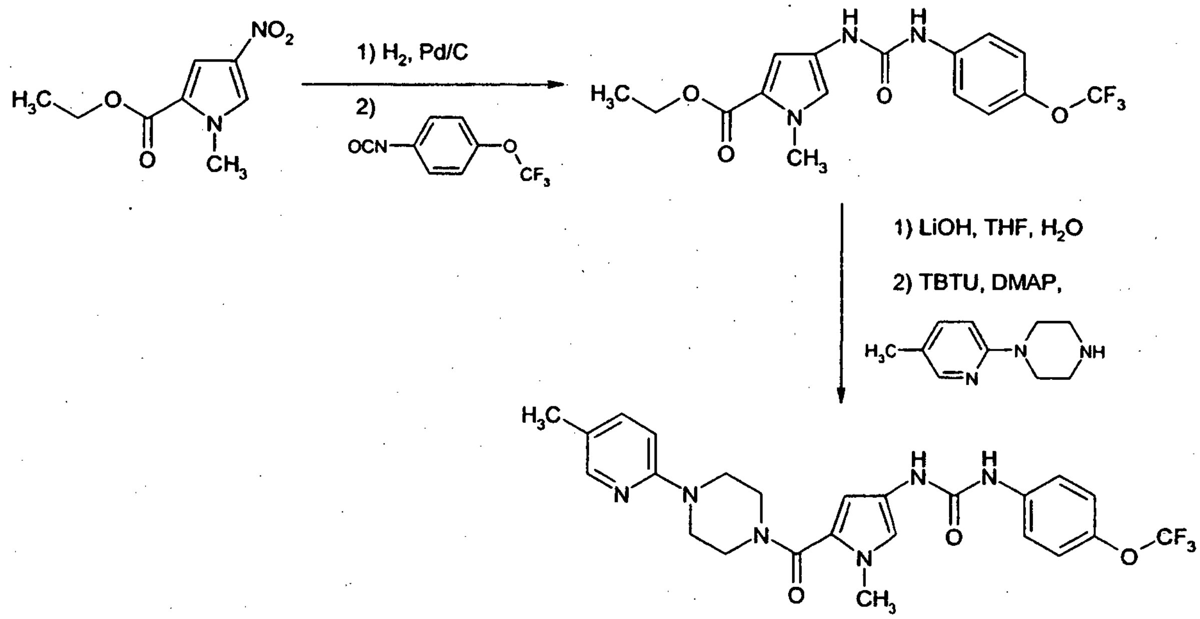


Syntheseschema 2:

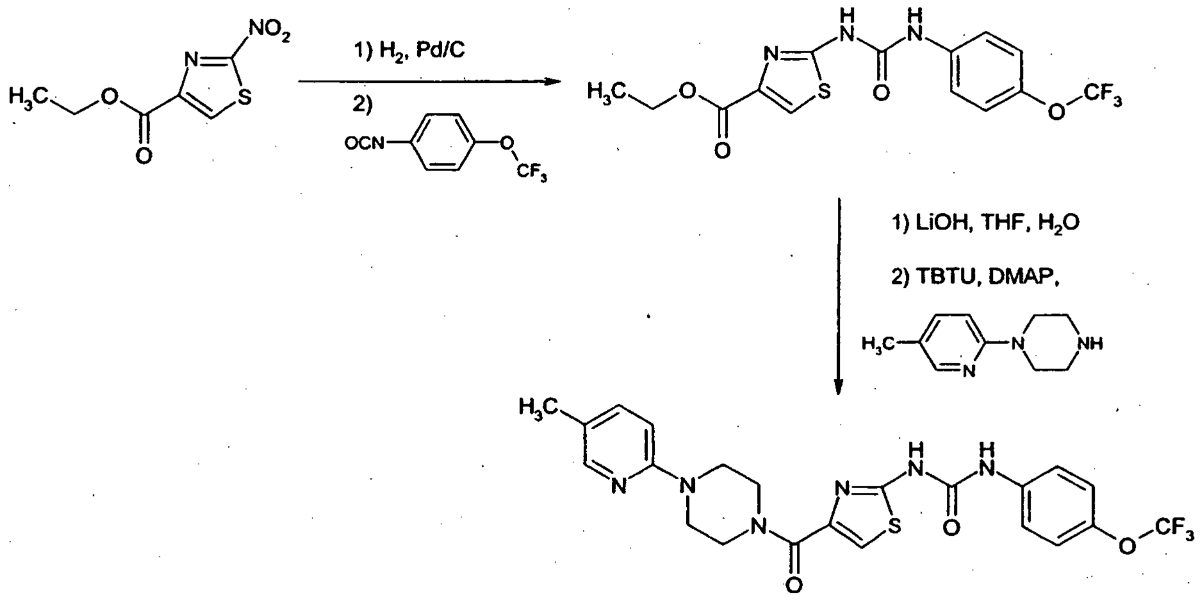


Die erfindungsgemäßen Verbindungen der allgemeinen Formel (I) zeigen ein nicht vorhersehbares, überraschendes Wirkspektrum. Sie zeigen eine antivirale Wirkung gegenüber Vertretern der Gruppe der Herpes viridae (Herpesviren), vor allem gegenüber Cytomegalieviren (CMV), insbesondere gegenüber dem humanen Cytomegalievirus (HCMV). Sie sind somit geeignet zur Behandlung und Prophylaxe von Krankheiten, vor allem von Infektionen mit Viren, insbesondere den vorstehend genannten Viren, und den dadurch hervorgerufenen Infektionskrankheiten. Unter einer Virusinfektion wird nachfolgend sowohl eine Infektion mit einem Virus als auch eine durch eine Infektion mit einem Virus hervorgerufene Krankheit verstanden.

Die Verbindungen der allgemeinen Formel (I) können aufgrund ihrer besonderen Eigenschaften zur Herstellung von Arzneimitteln, die zur Prophylaxe und/oder Behandlung von Krankheiten, insbesondere Virusinfektionen, geeignet sind, verwendet werden.

Als Indikationsgebiete können beispielsweise genannt werden:

1) Behandlung und Prophylaxe von HCMV-Infektionen bei AIDS-Patienten (Retinitis, Pneumonitis, gastrointestinale Infektionen).

2) Behandlung und Prophylaxe von Cytomegalievirus-Infektionen bei Knochenmark- und Organtransplantationspatienten, die an einer HCMV-Pneumonitis, -Enzephalitis, sowie an gastrointestinalen und systemischen HCMV-Infektionen oft lebensbedrohlich erkranken.

3) Behandlung und Prophylaxe von HCMV-Infektionen bei Neugeborenen und Kleinkindern.

4) Behandlung einer akuten HCMV-Infektion bei Schwangeren.

5) Behandlung der HCMV-Infektion bei immunsupprimierten Patienten bei Krebs und KrebsTherapie.

6) Behandlung von HCMV-positiven Krebspatienten mit dem Ziel, HCMV-vermittelte Tumorprogression zu verringern (vgl. J. Cinatl, et al., FEMS Microbiology Reviews 2004, 28, 59-77).

Bevorzugt werden die erfindungsgemäßen Verbindungen zur Herstellung von Arzneimitteln verwendet, die zur Prophylaxe und/oder Behandlung von Infektionen mit einem Vertreter der Gruppe der Herpes viridae, besonders einem Cytomegalievirus, insbesondere dem humanen Cytomegalie-Virus, geeignet sind.

Die erfindungsgemäßen Verbindungen können aufgrund ihrer pharmakologischen Eigenschaften allein und bei Bedarf auch in Kombination mit anderen Wirkstoffen, insbesondere antiviralen Wirkstoffen wie beispielsweise Ganciclovir, Valganciclovir oder Aciclovir, zur Behandlung und/oder Prävention von Virusinfektionen, insbesondere von HCMV-Infektionen, eingesetzt werden.

Weiterer Gegenstand der vorliegenden Erfindung ist der Einsatz der erfindungs gemäßen Verbindungen zur Behandlung und/oder Prophylaxe von Erkrankungen, vorzugsweise von Virus-infektionen, insbesondere von Infektionen mit dem humanen Cytomegalievirus (HCMV) oder einem anderen Vertreter der Gruppe der Herpes viridae.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Er-krankungen, insbesondere der zuvor genannten Erkrankungen.

Die erfindungsgemäßen Verwindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B, Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme, Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol),

Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei intravenöser Applikation Mengen von etwa 0.001 bis 10 mg/kg, vorzugsweise etwa 0.01 bis 5 mg/kg Körpergewicht zur Erzielung wirksamer Ergebnisse zu verabreichen, und bei oraler Applikation beträgt die Dosierung etwa 0.01 bis 25 mg/kg, vorzugsweise 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

## A. Beispiele

### Verwendete Abkürzungen:

| | |
|---|---|
| BINAP | 2,2'-Bis-(diphenylphosphino)-1,1'-binaphthyl |
| Bsp. | Beispiel |
| $CD_3CN$ | Deuteroacetonitril |
| DC | Dünnschichtchromatographie |
| DCI | direkte chemische Ionisation (bei MS) |
| DCM | Dichlormethan |
| DIEA | *N,N*-Diisopropylethylamin (Hünig Base) |
| DMAP | 4-*N,N*-Dimethylaminopyridin |
| DMF | *N,N*-Dimethylformamid |
| DMSO | Dimethylsulfoxid |
| d. Th. | der Theorie |
| EDCI x HCl | N'-(3-Dimethylaminopropyl)-N-ethylcarbodimid Hydrochlorid |
| EE | Ethylacetat (Essigsäureethylester) |
| EI | Elektronenstoß-Ionisation (bei MS) |
| ESI | Elektrospray-Ionisation (bei MS) |
| Fp. | Schmelzpunkt |
| ges. | gesättigt |
| h | Stunde |
| HATU | *O*-(7-Azabenzotriazol-1-yl)-*N,N,N',N'*-tetramethyluronium-hexafluorophosphat |
| HBTU | O-(Benzotriazol-1-yl)-*N,N,N',N'*-tetramethyluronium-hexafluorophosphat |
| HPLC | Hochdruck-, Hochleistungsflüssigchromatographie |
| i. V. | im Vakuum |
| konz. | konzentriert |
| LC-MS | Flüssigchromatographie-gekoppelte Massenspektroskopie |
| LDA | Lithiumdiisopropylamid |
| Lit. | Literatur(stelle) |
| Lsg. | Lösung |
| MS | Massenspektroskopie |
| NMR | Kernresonanzspektroskopie |
| proz. | prozentig |
| RP-HPLC | Reverse Phase HPLC |
| RT | Raumtemperatur |

| | |
|---|---|
| $R_t$ | Retentionszeit (bei HPLC) |
| Schmp. | Schmelzpunkt |
| TBTU | O-(Benzotriazol'-1-yl)-N,N,N',N'-tetramethyluronium-tetrafluoroborat |
| THF | Tetrahydrofuran |
| verd. | verdünnt |
| wässr. | wässrig |

**HPLC- und LC-MS-Methoden:**

**Methode 1 (LC-MS):** Gerätetyp MS: Micromass ZQ; Gerätetyp HPLC: Waters Alliance 2795; Säule: Phenomenex Synergi 2μ Hydro-RP Mercury 20 mm x 4 mm; Eluent A: 11 Wasser + 0.5 mL 50%ige Ameisensäure, Eluent B: 11 Acetonitril + 0.5 mL 50%ige Ameisensäure; Gradient: 0.0 min 90%A → 2.5 min 30%A → 3.0 min 5%A → 4.5 min 5%A; Fluss: 0.0 min 1 mL/min, 2.5 min/3.0 min/4.5 min 2 mL/min; Ofen: 50°C; UV-Detektion: 210 nm.

**Methode 2 (LC-MS):** Instrument: Micromass Quattro LCZ mit HPLC Agilent Serie 1100; Säule: Phenomenex Synergi 2μ Hydro-RP Mercury 20 mm x 4 mm; Eluent A: 11 Wasser + 0.5 mL 50%ige Ameisensäure, Eluent B: 11 Acetonitril + 0.5 mL 50%ige Ameisensäure; Gradient: 0.0 min 90%A → 2.5 min 30%A → 3.0 min 5%A → 4.5 min 5%A; Fluss: 0.0 min 1 mL/min, 2.5 min/3.0 min/4.5 min 2 mL/min; Ofen: 50°C; UV-Detektion: 208- 400 nm.

**Methode 3 (LC-MS):** Gerätetyp MS: Micromass ZQ; Gerätetyp HPLC: HP 1100 Series; UV DAD; Säule: Phenomenex Synergi 2μ Hydro-RP Mercury 20 mm x 4 mm; Eluent A: 11 Wasser + 0.5 mL 50%ige Ameisensäure, Eluent B: 11 Acetonitril + 0.5 mL 50%ige Ameisensäure; Gradient: 0.0 min 90%A → 2.5 min 30%A → 3.0 min 5%A → 4.5 min 5%A; Fluss: 0.0 min 1 mL/min, 2.5 min/3.0 min/4.5 min 2 mL/min; Ofen: 50°C; UV-Detektion: 210 nm.

**Ausgansverbindungen**

**Beispiel 1A**

1-Methyl-2-trichloracetyl-1H-pyrrol

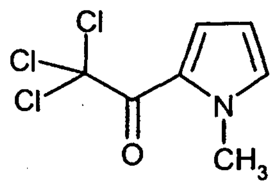

1.09 mL (12.3 mmol) Trichloracetylchlorid werden unter Argon in 5 mL DCM vorgelegt und eine Lösung von N-Methylimidazol in 3 mL DCM bei RT innerhalb von 30 min zugetropft. Man lässt über Nacht bei RT rühren, engt die Reaktionslösung ein und reinigt den Rückstand über eine Flashfritte (Cyclohexan, Cyclohexan/Ethylacetat 40:1). Man erhält das Produkt als eine Flüssigkeit.
Ausbeute: 2.12 g (76% d. Th.)
LC-MS (Methode 1): $R_t$ = 2.34 min.; MS ($EI^+$): m/z = 225 ($M^+$)

**Beispiel 2A**

1-Methyl-4-nitro-2-trichloracetyl-1H-pyrrol

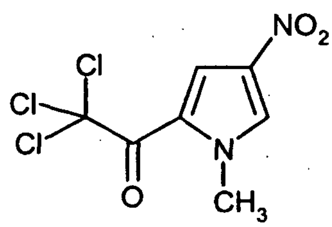

2.12 g (9.34 mmol) 1-Methyl-2-trichloracetyl-1H-pyrrol werden in 9.5 mL Acetanhydrid gelöst, auf -20°C gekühlt und mit 0.43 mL (9.34 mmol) Salpetersäure versetzt. Man lässt langsam auf RT erwärmen rührt noch 1 h bei RT. Das Reaktionsgemisch wird auf 95 g Eis gegossen und 2.5 h kräftig gerührt (zunächst ölige Abscheidung, dann Kristallisation). Der Niederschlag wird abgesaugt, mit 20 mL Methanol verrührt, filtriert und über Nacht im Vakuum getrocknet. Zum Entfernen des ebenfalls entstandenen Regioisomeren wird das Gemisch 2 h mit 10 mL Essigsäure/Wasser 1:1 verrührt, der Feststoff abgesaugt und im Vakuum getrocknet. Man erhält einen Feststoff.
Ausbeute: 1.71 g (67% d. Th.)
LC-MS (Methode 2): $R_t$ = 2.51 min.
$^1$H-NMR (400MHz, DMSO-$d_6$): $\delta$ = 8.58 (d, 1H), 7.80 (d, 1H), 4.00 (s, 3H).

**Beispiel 3A**

1-Methyl-4-nitro-1H-pyrrol-2-carbonsäureethylester

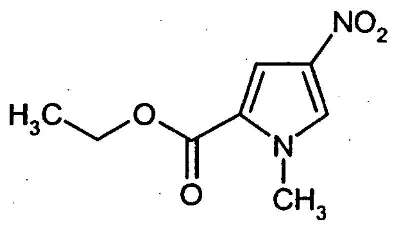

0.50 g (1.84 mmol) 1-Methyl-4-nitro-2-trichloracetyl-1H-pyrrol werden in 5 mL Ethanol vorgelegt, mit 0.26 mL (1.84 mmol) Triethylamin versetzt und bei RT 2 h gerührt. Das Reaktionsgemisch wird mit 5 mL Wasser versetzt, 30 min bei 0°C gerührt und anschließend der Niederschlag abgesaugt und im Vakuum getrocknet.
Ausbeute: 321 mg (88% d. Th.)
LC-MS (Methode 3): $R_t$ = 2.25 min.; MS (ESI$^+$): m/z = 199 $(M+H)^+$
$^1$H-NMR (300MHz, DMSO-$d_6$): $\delta$ = 8.29 (d, 1H), 7.31 (d, 1H), 4.27 (q, 2H), 3.92 (s, 3H), 1.30 (t, 3H).

**Beispiel 4A**

1-Methyl-4-[({[4-(trifluormethoxy)phenyl]amino}carbonyl)amino]-1H-pyrrol-2-carbonsäure-ethylester

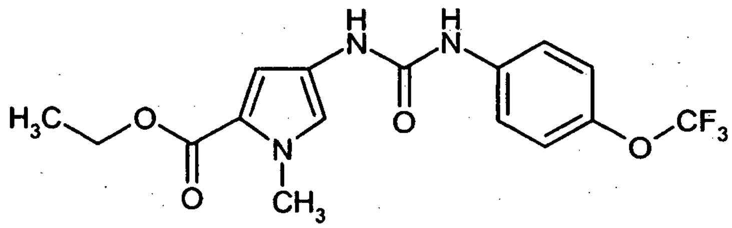

304mg (1.53 mmol) 1-Methyl-4-nitro-1H-pyrrol-2-carbonsäureethylester werden in 6 mL Ethylacetat/Ethanol (1:1) vorgelegt, mit 163 mg (0.15 mmol) Palladium (10%ig auf Aktivkohle) und 580 mg (9.20 mmol) Ammoniumformiat versetzt und 1 h bei 80°C gerührt. Nach dem Abkühlen wird über Kieselgur filtriert, mit Ethanol nachgespült und das Filtrat im Vakuum vom Lösungsmittel befreit. Der Rückstand wird in 6 mL THF gelöst, mit 374 mg (1.84 mmol) 4-Triluormethoxyphenylisocyanat versetzt und 1 h bei RT gerührt. Die Reaktionslösung. wird eingeengt und der Rückstand durch RP-HPLC (Acetonitril/Wasser) gereinigt. Man erhält einen Feststoff.
Ausbeute: 486 mg (85% d. Th.)
LC-MS (Methode 3): $R_t$ = 2.61 min.; MS (ESI$^+$): m/z = 372 $(M+H)^+$
$^1$H-NMR (300MHz, DMSO-$d_6$): $\delta$ = 8.80 (s, 1H), 8.39 (s, 1H), 7.53 (m, 2H), 7.26 (d, 2H), 7.20 (d, 1H), 6.72 (d, 1H), 4.20 (q, 2H), 3.82 (s, 3H), 1.28 (t, 3H).

**Beispiel 5A**

1-Methyl-4-[({[4-(trifluormethoxy)phenyl]amino}carbonyl)amino]-1H-pyrrol-2-carbonsäure

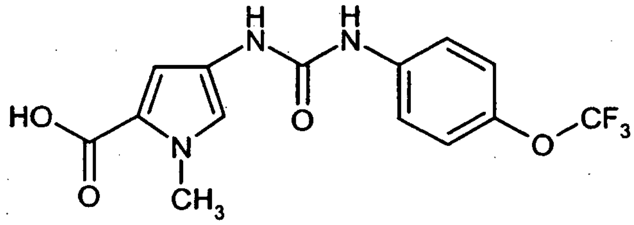

470 mg (1.27 mmol) 1-Methyl-4-[({[4-(trifluormethoxy)phenyl]amino}carbonyl)amino]-1H-pyrrol-2-carbonsäureethylester werden in 5 mL THF vorgelegt, 152 mg (6.33 mmol) Lithiumhydroxid in 1 mL Wasser zugegeben und über Nacht unter Rückfluss gerührt. Das Reaktionsgemisch wird eingeengt, der Rückstand mit 2M Salzsäure angesäuert und der entstandene Niederschlag abgesaugt und im Vakuum getrocknet. Man erhält einen Feststoff.
Ausbeute: 429 mg (98% d. Th.)
LC-MS (Methode 2): $R_t$ = 2.09 min.; MS (ESI$^+$): m/z = 344 (M+H)$^+$
$^1$H-NMR (300MHz, DMSO-$d_6$): $\delta$ = 12.16 (bs, 1H), 9.01 (s, 1H), 8.58 (s, 1H), 7.53 (m, 2H), 7.25 (d, 2H), 7.18 (d, 1H), 6.63 (d, 1H), 3.80 (s, 3H).

**Beispiel 6A**

1-(5-Methylpyridin-2-yl)piperazin

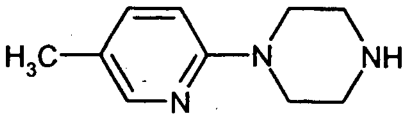

Stufe 1

1-(tert-Butyloxycarbonyl)-4-(5-methylpyridin-2=yl)piperazin

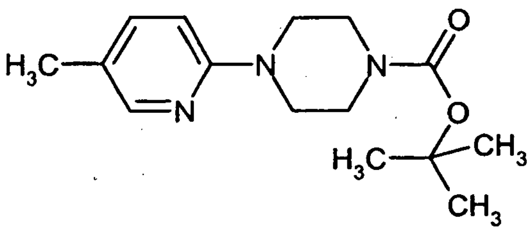

Unter einer Argonatmosphäre werden 2.50 g (19.6 mmol) 2-Methyl-5-chlorpyridin und 4.38 g (23.5 mmol) N-(tert-Butyloxycarbonyl)-piperazin in 50 mL absolutem Toluol gelöst. Anschließend gibt man 2.26 g (23.5 mmol) Natrium-tert-butylat, 0.37 g (0.59 mmol) BINAP und 0.36 g (0.39 mmol) Tris(dibenzylidenaceton)dipalladium hinzu und erhitzt 12 h auf 70°C. Nach dem Abkühlen wird das Reaktionsgemisch mit Diethylether versetzt, dreimal mit gesättigter Natriumchloridlösung gewaschen, über Natriumsulfat getrocknet und im Vakuum vom Lösungsmittel befreit. Der Rückstand wurde flashchromatographisch (Cyclohexan/Ethylacetat 9:1) gereinigt.
Ausbeute: 5.27 g (97% d. Th.).
LC-MS (Methode 1): $R_t$ = 1.26 min.; MS (ESI$^+$): m/z = 278 (M+H)$^+$

$^1$H-NMR (300MHz, $CDCl_3$): δ = 8.02 (d, 1H), 7.34 (dd, 1H), 6.59 (d, 1H), 3.55 (m, 4H), 3.45 (m, 4H), 2.21 (s, 3H), 1.49 (s, 9H).

Stufe 2

1-(5-Methylpyridin-2-yl)piperazin

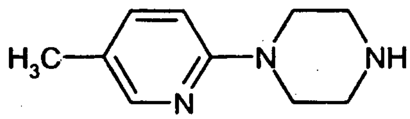

3.47 g (12.5 mmol) 1-(tert-Butyloxycarbonyl)-4-(S-methylpyridin-2-yl)piperazin werden in 10 mL Dioxan gelöst und mit 31 mL (125 mmol) Chlorwasserstoff in Dioxan (4 molar) versetzt. Man lässt 2 h bei RT rühren. Anschließend wird eingeengt, der Rückstand mit 1M Natronlauge alkalisiert und mehrmals mit Dichlormethan extrahiert. Die vereinigten organischen Phasen werden über Natriumsulfat getrocknet, eingeengt und im Vakuum getrocknet.
Ausbeute: 2.18 g (98% d. Th.).
LC-MS (Methode 3): $R_t$ = 0.38 min.; MS (ESI$^+$): m/z = 177 $(M+H)^+$
$^1$H-NMR (300MHz, $CDCl_3$): δ = 8.02 (d, 1H), 7.32 (dd, 1H), 6.59 (d, 1H), 3.45 (m, 4H), 3.00 (m, 4H), 2.20 (s, 3H).

**Beispiel 7A**

1-Ethyl-4-[({[4-(trifluormethoxy)phenyl]amino}carbonyl)amino]-1H-pyrrol-2-carbonsäure

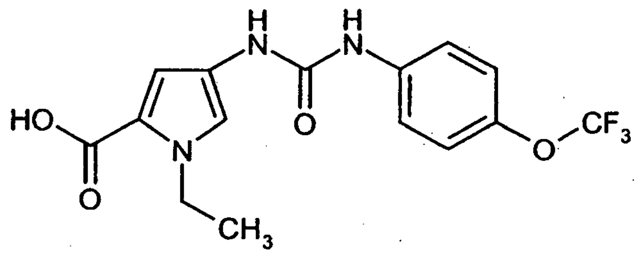

Die Herstellung erfolgt analog zu Beispiel 5A.
LC-MS (Methode 2): $R_t$ = 2.10 min.; MS (ESI$^+$): m/z = 358 $(M+H)^+$

**Beispiel 8A**

1-Butyl-4-[({[4-(trifluormethoxy)phenyl]amino}carbonyl)amino]-1H-pyrrol-2-carbonsäure

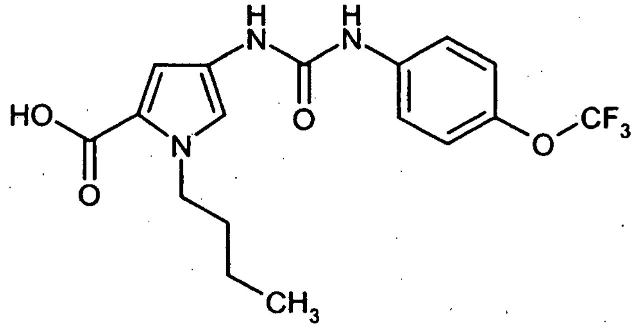

Die Herstellung erfolgt analog zu Beispiel 5A.
LC-MS (Methode 3): $R_t$ = 2.47 min.; MS ($ESI^+$): m/z = 386 $(M+H)^+$
$^1$H-NMR (300MHz, DMSO-$d_6$): δ = 8.90 (bs, 1H), 8.48 (bs, 1H), 7.54 (d, 2H), 7.26 (d, 2H), 7.24 (d, 1H), 6.73 (d, 1H), 4.21 (t, 2H), 1.62 (quint., 2H), 1.25 (sext., 2H), 0.88 (t, 3H).

**Beispiel 9A**

1-(Cyclopropylmethyl)-4-[{[4-(trifluormethoxy)phenyl]amino}carbonyl)amino]-1H-pyrrol-2-carbonsäure

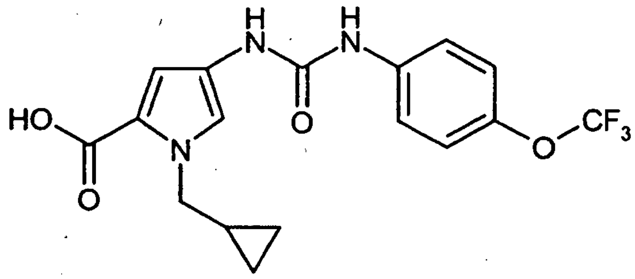

Die Herstellung erfolgt analog zu Beispiel 5A.
LC-MS (Methode 2): $R_t$ = 2.33 min.; MS ($ESI^+$): m/z = 384 $(M+H)^+$
$^1$H-NMR (300MHz, DMSO-$d_6$): δ = 8.86 (bs, 1H), 8.45 (bs, 1H), 7.55 (m, 2H), 7.23-7.32 (m, 3H), 6.68 (d, 1H), 4.11 (d, 2H), 1.21 (m, 1H), 0.45 (m, 2H), 0.32 (m, 2H).

**Beispiel 10A**

4-[({[4-(Trifluormethoxy)phenyl]amino}carbonyl)amino]thiophen-2-carbonsäure

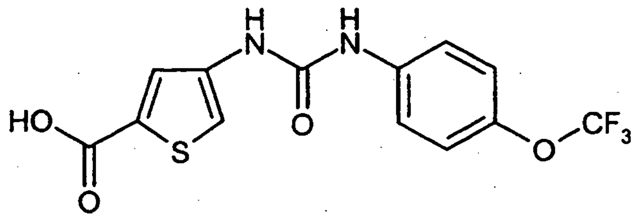

Die Herstellung erfolgt analog zu Beispiel 4A und 5A ausgehend von 4-Aminothiophen-2-carbonsäuremethylester (synthetisiert nach A. A. Kiryano et al., *Tetrahedron Lett.* **2001,** (42), 8797-8800).
Ausbeute: 72 mg (27% d. Th., 2 Stufen)
LC-MS (Methode 2): $R_t$ = 2.25 min.; MS ($ESI^+$): m/z = 347 $(M+H)^+$
$^1$H-NMR (400MHz, DMSO-$d_6$): δ = 13.1 (bs, 1H), 9.10 (bs, 1H), 8.98 (bs, 1H), 7.69 (s, 1H), 7.53-7.60 (m, 3H), 7.29 (d, 2H).

**Beispiel 11A**

2-[({[4-(Trifluormethoxy)phenyl]amino}carbonyl)amino]-1,3-thiazol-4-carbonsäure

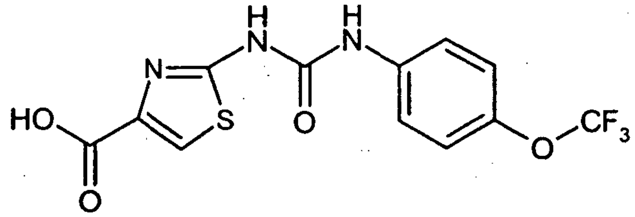

Die Herstellung erfolgt analog zu Beispiel 4A, und 5A ausgehend von 2-Amino-1,3-thiazol-4-carbonsäureethylester (kommerziell erhältlich bei ACROS).
Ausbeute: 290 mg (61% d. Th., 2 Stufen)
LC-MS (Methode 2): $R_t$ = 2.05 min.; MS (ESI$^+$): m/z = 348 $(M+H)^+$
$^1$H-NMR (300MHz, DMSO-$d_6$): $\delta$ = 12.8 (bs, 1H), 10.9 (bs, 1H), 9.22 (bs, 1H), 7.91 (s, 1H), 7.60 (d, 2H), 7.32 (d, 2H).

## Beispiel 12A

5-Methyl-2-[({[4-(trifluormethoxy)phenyl]amino}carbonyl)amino]-1,3-thiazol-4-carbonsäure

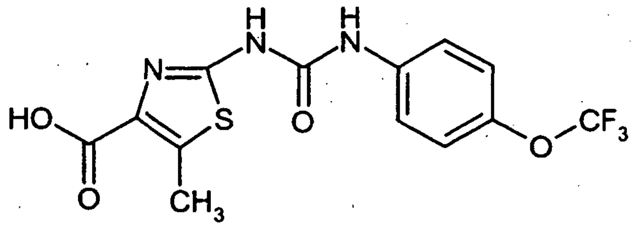

Die Herstellung erfolgt analog zu Beispiel 4A und 5A ausgehend von 2-Amino-5-methyl-1,3-thiazol-4-carbonsäuremethylester (kommerziell erhältlich bei Tyger Scientific).
Ausbeute: 148 mg (40% d. Th., 2 Stufen)
LC-MS (Methode 3): $R_t$ = 2.47 min.; MS (ESI$^+$): m/z = 362 $(M+H)^+$
$^1$H-NMR (300MHz, DMSO-$d_6$): $\delta$ = 12.7 (bs, 1H), 10.7 (bs, 1H), 9.18 (bs, 1H), 7.59 (d, 2H), 7.32 (d, 2H), 3.34 (s, 3H).

## Beispiel 13A

5-Chlor-2-[({[4-(trifluormethoxy)phenyl]amino}carbonyl)amino]-1,3-thiazol-4-carbonsäure

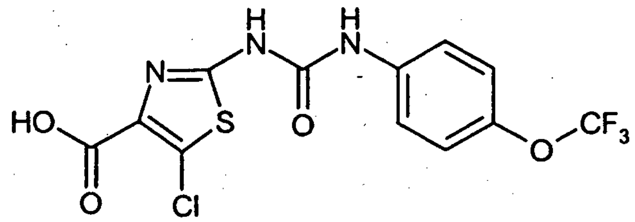

Die Herstellung erfolgt analog zu Beispiel 4A und 5A ausgehend von 2-Amino-5-chlor-1,3-thiazol-4-carbonsäureethylester (Synthese beschrieben in K. J. Hodgetts et. al., *Org. Lett.* **2002,** (4), 1363-1366).
Ausbeute: 365 mg (89% d. Th., 2 Stufen)
LC-MS (Methode 3): $R_t$ = 2.51 min.; MS (ESI$^+$): m/z = 382 $(M+H)^+$
$^1$H-NMR (300MHz, DMSO-$d_6$): $\delta$ = 13.2 (bs, 1H), 11.2 (bs, 1H), 9.28 (bs, 1H), 7.58 (m, 2H), 7.33 (m, 2H).

**Beispiel 14A**

5-Brom-2-[({[4-(trifluormethoxy)phenyl]amino}carbonyl)amino]-1,3-thiazol-4-carbonsäure

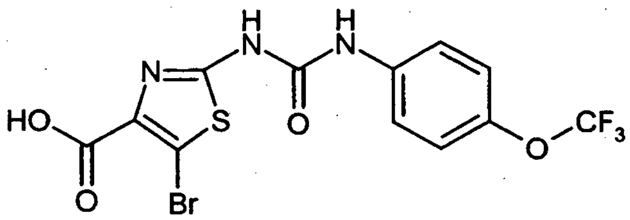

Die Herstellung erfolgt analog zu Beispiel 4A und 5A ausgehend von 2-Amino-5-brom-1,3-thiazol-4-carbonsäureethylester (Synthese beschrieben in J. F. Okonya et. al., *Tetrahedron Lett.,* **2002,** (43), 7051-7054).
Ausbeute: 343 mg (74% d. Th., 2 Stufen)
LC-MS (Methode 2): $R_t$ = 2.22 min.; MS ($ESI^+$): m/z = 426 $(M+H)^+$
$^1$H-NMR (300MHz, DMSO-$d_6$): $\delta$ = 13.1 (bs, 1H), 11.2 (bs, 1H), 9.28 (bs, 1H), 7.58 (m, 2H), 7.32 (m, 2H).

**Beispiel 15A**

2-[({[4-(Trifluormethoxy)phenyl]amino}carbonyl)amino]-1,3-thiazol-5-carbonsäure

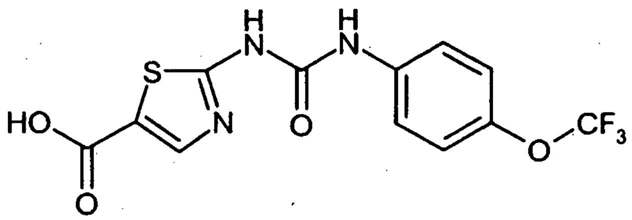

Die Herstellung erfolgt analog zu Beispiel 4A und 5A ausgehend von 2-Amino-1,3-thiazol-5-carbonsäureethylester (kommerziell erhältlich bei RareChem).
Ausbeute: 200 mg (55% d. Th., 2 Stufen)
LC-MS (Methode 3): $R_t$ = 2.36 min.; MS ($ESI^+$): m/z = 348 $(M+H)^+$
$^1$H-NMR (300MHz, DMSO-$d_6$): $\delta$ = 12.8 (bs, 1H), 10.9 (bs, 1H), 9.21 (bs, 1H), 7.92 (s, 1H), 7.60 (d, 2H), 7.33 (d, 2H).

**Ausführungsbeispiele**

**Beispiel 1**

N-(1-Methyl-5-[(4-pyridin-2-ylpiperazin-1-yl)carbonyl]-1H-pyrrol-3-yl)-N'-[4-(trifluormethoxy)-phenyl]harnstoff

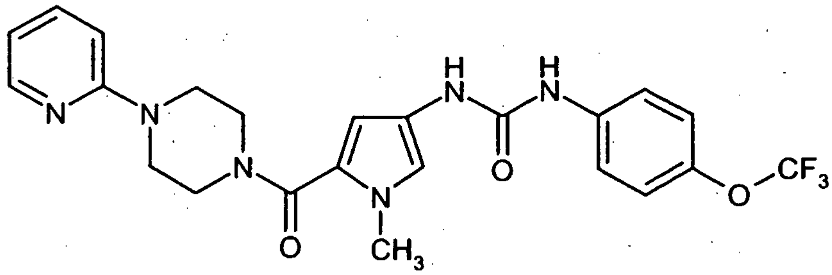

50 mg (0.15 mmol) 1-Methyl-4-[({[4-(trifluormethoxy)phenyl]amino}carbonyl)amino]-1H-pyrrol-2-carbonsäure (Beispiel 5A) werden in 1 mL DMF vorgelegt und mit 56 mg (0.18 mmol) O-(Benzotriazol-1-yl)-N,N,N',N'-tetramethyluroniumtetrafluoroborat (TBTU) sowie 8.9 mg (0.07 mmol) 4-(Dimethylamino)-pyridin (DMAP) versetzt. Anschließend gibt man 29 mg (0.18 mmol) 1-(2-Pyridyl)-piperazin hinzu und rührt 8 h bei RT. Die Reaktionslösung wird durch RP-HPLC (Acetonitril/Wasser) gereinigt. Man erhält einen Feststoff.
Ausbeute: 54 mg (76% d. Th.)
LC-MS (Methode 1): $R_t$ = 1.61 min., MS (ESI$^+$): m/z = 489 $(M+H)^+$
$^1$H-NMR (300MHz, DMSO-$d_6$): $\delta$ = 8.75 (s, 1H), 8.32 (s, 1H), 8.13 (dd, 1H), 7.50-7.59 (m, 3H), 7.25 (d, 2H), 7.02 (d, 1H), 6.86 (d, 1H), 6.68 (dd, 1H), 6.28 (d, 1H), 3.72 (m, 4H), 3.64 (s, 3H), 3.55 (m, 4H).

**Beispiel 2**

N-(1-Methyl-5-{[4-(5-methylpyridin-2-yl)piperazin-1-yl]carbonyl}-1H-pyrrol-3-yl)-N'-[4-(trifluormethoxy)phenyl]hamstoff

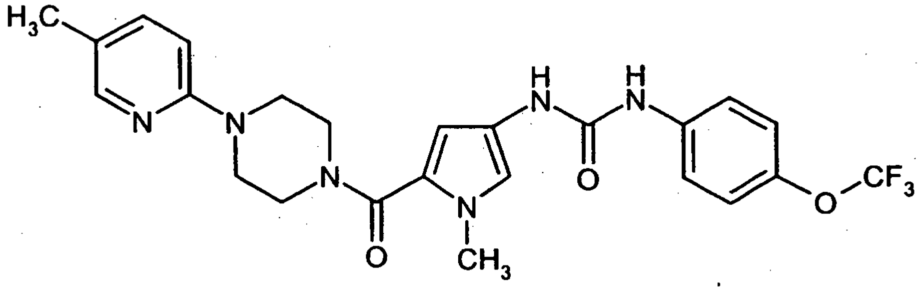

Die Herstellung erfolgt analog zu Beispiel 1 aus Beispiel 5A und 6A.
Ausbeute: 50 mg (68% d. Th.)
LC-MS (Methode 1): $R_t$ = 1.66 min.; MS (ESI$^+$): m/z = 503 $(M+H)^+$
$^1$H-NMR (300MHz, DMSO-$d_6$): $\delta$ = 8.75 (bs, 1H), 8.32 (bs, 1H), 7.98 (d, 1H), 7.53 (m, 2H), 7.42 (dd, 1), 7.25 (d, 2H), 7.02 (d, 1H), 6.80 (d, 1H), 6.28 (d, 1H), 3.68-3.76 (m, 4H), 3.63 (s, 3H), 3.45-3.53 (m, 4H), 2.17 (s, 3H).

**Beisuiel 3**

N-{1-Ethyl-5-[(4-pyridin-2-ylpiperazin-l-yl)carbonyl]-1H-pyrrol-3-yl}-N'-[4-(trifluormethoxy)-phenyl]harnstoff

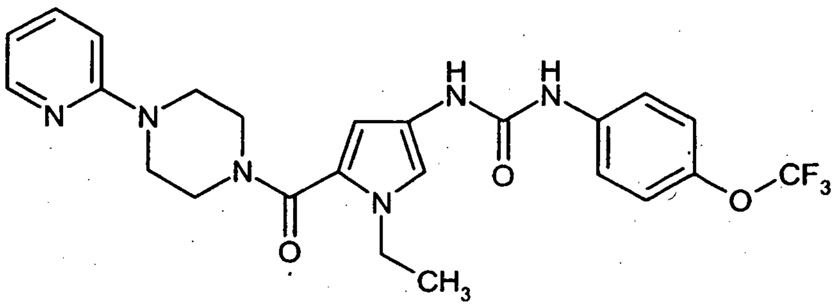

Die Herstellung erfolgt analog zu Beispiel 1 aus Beispiel 7A und 6A.
Ausbeute: 29 mg (43% d. Th.)
LC-MS (Methode 2): $R_t$ = 1.79 min.; MS (ESI$^+$): m/z = 503 $(M+H)^+$
$^1$H-NMR (400MHz, DMSO-$d_6$): $\delta$ = 8.74 (bs, 1H), 8.32 (bs, 1H), 8.13 (d, 1H), 7.49-7.60 (m, 3H), 7.22-7.28 (m, 2H), 7.08 (s, 1H), 6.87 (d, 1H), 6.68 (dd, 1H), 6.26 (s, 1H), 4.04 (q, 2H), 3.68-3.77 (m, 4H), 3.50-3.58 (m, 4H), 1.26 (t, 3H).

**Beispiel 4**

N-(1-Ethyl-5-{[4-(5-methylpyridin-2-yl)piperazin-l-yl]carbonyl}-1H-pyrrol-3-yl)-N'-[4-(trifluormethoxy)phenyl]harnstoff

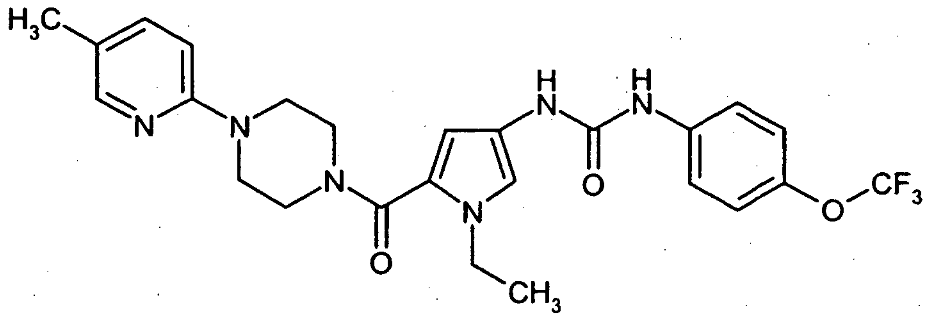

Die Herstellung erfolgt analog zu Beispiel 1 aus Beispiel 7A und 6A.
Ausbeute: 26 mg (39% d. Th.)
LC-MS (Methode 2): $R_t$ = 1.80 min.; MS (ESI$^+$): m/z = 517 (M+H)$^+$
$^1$H-NMR (400MHz, DMSO-$d_6$): $\delta$ = 8.75 (bs, 1H), 8.32 (bs, 1H), 7.98 (m, 1H), 7.54 (m, 2H), 7.42 (m, 1H), 7.26 (m, 2H), 7.08 (m, 1H), 6.80 (d, 1H), 6.25 (s, 1H), 4.03 (q, 2H), 3.68-3.76 (m, 4H), 3.44-3.52 (m, 4H), 2.18 (s, 3H), 1.25 (t, 3H).

**Beispiel 5**

N-{(1-Butyl-5-[(4-pyridin-2-ylpiperazin-1-yl)carbonyl]-1H-pyrrol-3-yl)}-N'-[4-(trifluormethoxy)-phenyl]harnstoff

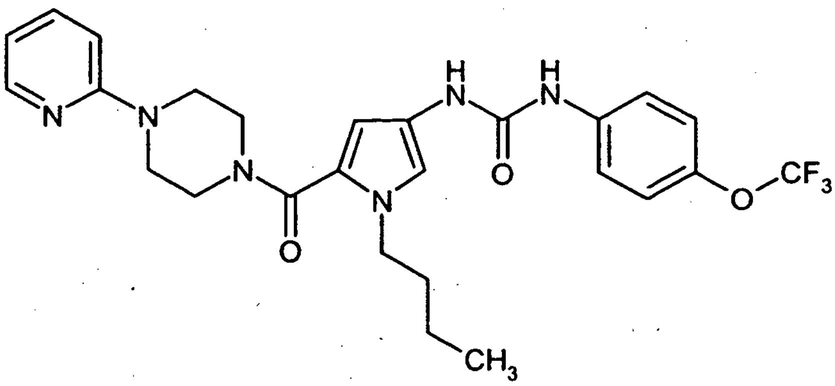

Die Herstellung erfolgt analog zu Beispiel 1 aus Beispiel 8A.
Ausbeute: 41 mg (69% d. Th.)
LC-MS (Methode 2): $R_t$ = 2.11 min.; MS (ESI$^+$): m/z = 531 (M+H)$^+$
$^1$H-NMR (400MHz, DMSO-$d_6$): $\delta$ = 8.75 (bs, 1H), 8.33 (bs, 1H), 8.12 (d, 1H), 7.58 (m, 1H), 7.54 (d, 2H), 7.26 (d, 2H), 7.07 (d, 1H), 6.87 (d, 1H), 6.68 (dd, 1H), 6.26 (d, 1H), 4.02 (t, 2H), 3.72 (m, 4H), 3.53 (m, 4H), 1.59 (quint., 2H), 1.18 (sext., 2H), 0.84 (t, 3H).

**Beispiel 6**

N-(1-Butyl-5-{[4-(5-methylpyridin-2-yl)piperazin-1-yl]carbonyl}-1H-pyrrol-3-yl)-N'-[4-(trifluormethoxy)phenyl]harnstoff

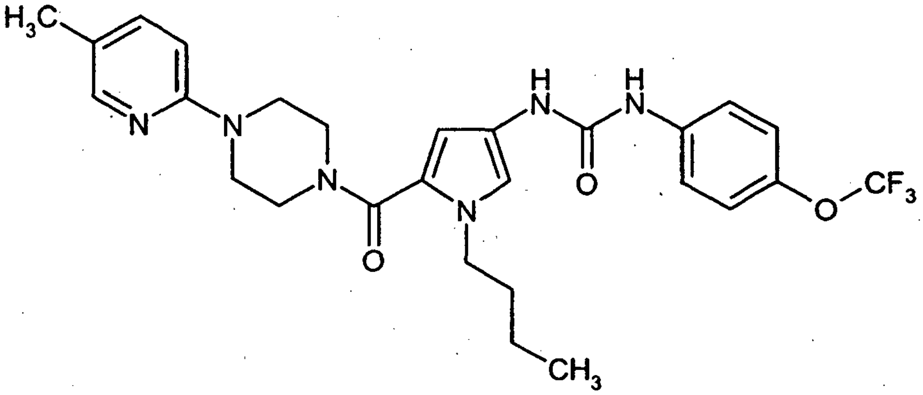

Die Herstellung erfolgt analog zu Beispiel 1 aus Beispiel 8A und 6A.
Ausbeute: 19 mg (20% d. Th.)
LC-MS (Methode 2): $R_t$ = 1.97 min.; MS ($ESI^+$): m/z = 545 $(M+H)^+$
$^1$H-NMR (300MHz, DMSO-$d_6$): $\delta$ = 8.74 (bs, 1H), 8.32 (bs, 1H), 7.98 (d, 1H), 7.53 (d, 2H), 7.41 (dd, 1H), 7.25 (d, 2H), 7.07 (d, 1H), 6.80 (d, 1H), 6.25 (d, 1H), 4.02 (t, 2H), 3.72 (m, 4H), 3.47 (m, 4H), 2.16 (s, 3H), 1.59 (quint., 2H), 1.18 (sext., 2H), 0.83 (t, 3H).

**Beispiel 7**

N-{1-(Cyclopropylmethyl)-5-[(4-pyridin-2-ylpiperazin-1-yl)carbonyl]-1H-pyrrol-3-yl}-N'-[4-(trifluormethoxy)phenyl]harnstoff

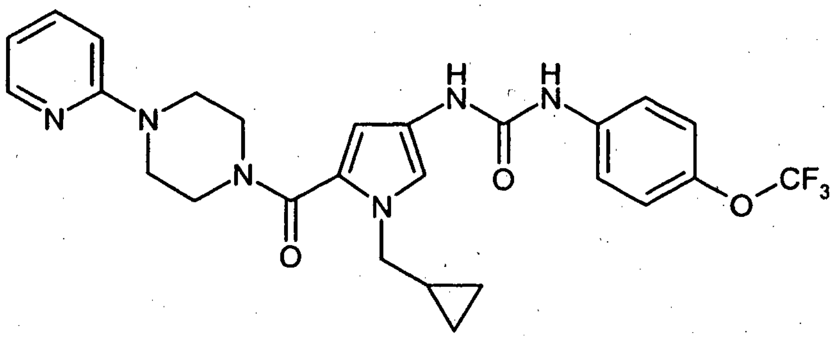

Die Herstellung erfolgt analog zu Beispiel 1 aus Beispiel 9A.
Ausbeute: 51 mg (86% d. Th.)
LC-MS (Methode 1): $R_t$ = 1.84 min.; MS ($ESI^+$): m/z = 529 $(M+H)^+$
$^1$H-NMR (300MHz, DMSO-$d_6$): $\delta$ = 8.75 (bs, 1H), 8.33 (bs, 1H), 8.13 (dt, 1H), 7.51-7.61 (m, 3H), 7.27 (d, 2H), 7.13 (d, 1H), 6.88 (d, 1H), 6.69 (dd, 1H), 6.28 (d, 1H), 3.90 (d, 2H), 3.73 (m, 4H), 3.53 (m, 4H), 1.12 (m, 1H), 0.45 (m, 2H), 0.28 (m, 2H).

**Beispiel 8**

N-(1-(Cyclopropylmethyl)-5-{[4-(5-methylpyridin-2-yl)piperazin-1-yl]carbonyl}-1H-pyrrol-3-yl)-N'-[4-(trifluormethoxy)phenyl]harnstoff

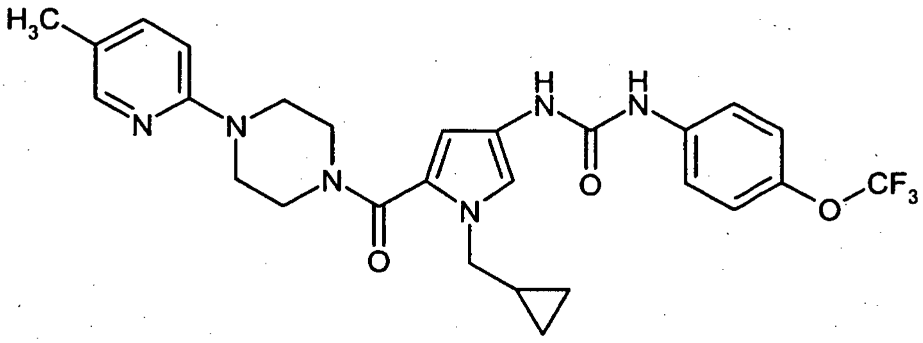

Die Herstellung erfolgt analog zu Beispiel 1 aus Beispiel 9A und 6A.
Ausbeute: 52 mg (85% d. Th.)
LC-MS (Methode 1): $R_t$ = 1.86 min.; MS (ESI$^+$): m/z = 543 $(M+H)^+$
$^1$H-NMR (300MHz, DMSO-$d_6$): $\delta$ = 8.77 (bs, 1H), 8.34 (bs, 1H), 7.98 (d, 1H), 7.54 (d, 2H), 7.41 (dd, 1H), 7.25 (d, 2H), 7.12 (d, 1H), 6.80 (d, 1H), 6.27 (d, 1H), 3.89 (d, 2H), 3.72 (m, 4H), 3.48 (m, 4H), 2.16 (s, 3H), 1.12 (m, 1H), 0.45 (m, 2H), 0.28 (m, 2H).

## Beispiel 9

N-(5-{[4-(5-Methylpyridin-2-yl)piperazin-1-yl]carbonyl}-3-thienyl)-N'-[4-(trifluormethoxy)-phenyl]harnstoff

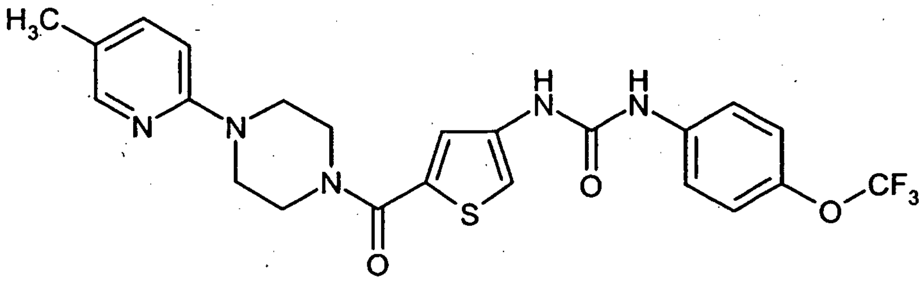

Die Herstellung erfolgt analog zu Beispiel 1 aus Beispiel 10A und 6A.
Ausbeute: 60 mg (63% d. Th.)
LC-MS (Methode 3): $R_t$ = 2.05 min.; MS (ESI$^+$): m/z = 506 $(M+H)^+$
$^1$H-NMR (400MHz, DMSO-$d_6$): $\delta$ = 9.05 (bs, 1H), 8.95 (bs, 1H), 7.98 (d, 1H), 7.56 (d, 2H), 7.47 (s, 1H), 7.39-7.44 (m, 2H), 7.29 (d, 2H), 6.80 (d, 1H), 3.75 (m, 4H), 3.51 (m, 4H), 2.16 (s, 3H).

Folgende Verbindungen werden analog zu Beispiel 1 aus den entsprechenden Ausgangsverbindungen synthetisiert:

| Bsp.-Nr. | Struktur | Ausgangsverbindung | Ausbeute | LC-MS $R_t$ [min] | MS $(M+H)^+$ |
|---|---|---|---|---|---|
| 10 | 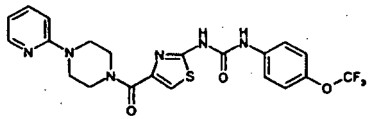 | Beispiel 11A | 36 mg (51% d. Th.) | 1.95 (Methode 3) | 493 |
| 11 | 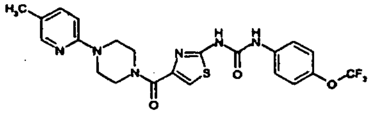 | Beispiel 11A | 49 mg (67% d. Th.) | 2.02 (Methode 3) | 507 |
| 12 | 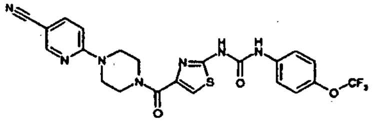 | Beispiel 11A | 43 mg (58% d. Th.) | 2.52 (Methode 3) | 518 |
| 13 | 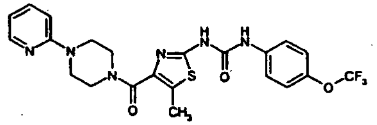 | Beispiel 12A | 33 mg (48% d. Th.) | 2.00 (Methode 3) | 507 |

| Bsp.-Nr. | Struktur | Ausgangsverbindung | Ausbeute | LC-MS $R_t$ [min] | MS $(M+H)^+$ |
|---|---|---|---|---|---|
| 14 | 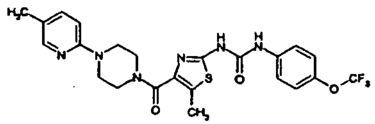 | Beispiel 12A | 50 mg (70% d. Th.) | 2.01 (Methode 3) | 521 |
| 15 | 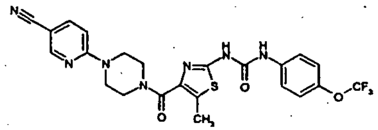 | Beispiel 12A | 52 mg (71% d. Th.) | 2.60 (Methode 3) | 532 |
| 16 | 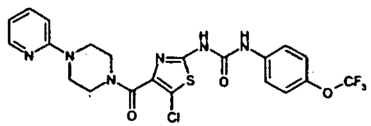 | Beispiel 13A | 43 mg (58% d. Th.) | 2.21 (Methode 3) | 527 |
| 17 | 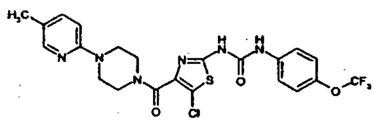 | Beispiel 13A | 35 mg (49% d. Th.) | 2.24 (Methode 3) | 541 |
| 18 | 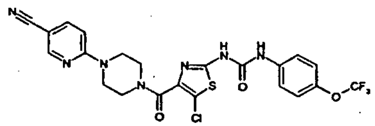 | Beispiel 13A | 42 mg (52% d. Th.) | 2.76 (Methode 3) | 552 |
| 19 | 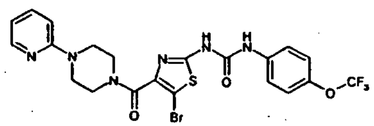 | Beispiel 14A | 45 mg (59% d. Th.) | 2.22 (Methode 3) | 571 |
| 20 | 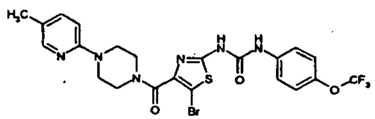 | Beispiel 14A | 50 mg (68% d. Th.) | 2.27 (Methode 3) | 585 |
| 21 | 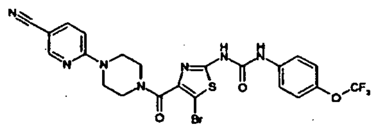 | Beispiel 14A | 41 mg (50% d. Th.) | 2.77 (Methode 3) | 596 |

(fortgesetzt)

| Bsp.-Nr. | Struktur | Ausgangsverbindung | Ausbeute | LC-MS $R_t$ [min] | MS $(M+H)^+$ |
|---|---|---|---|---|---|
| 22 | 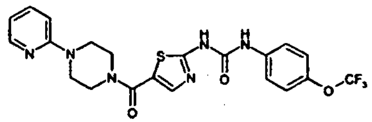 | Beispiel 15A | 34 mg (49% d. Th.) | 1.90 (Methode 3) | 493 |
| 23 | 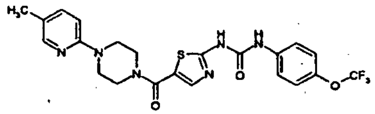 | Beispiel 15A | 47 mg (65% d. Th.) | 1.93 (Methode 3) | 507 |
| 24 | 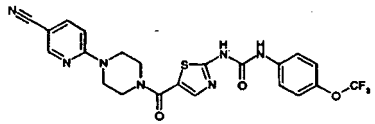 | Beispiel 15A | 47 mg (64% d. Th.) | 2.53 (Methode 3) | 518 |

**B. Bewertung der physiolozischen Wirksamkeit**

Die *in vitro*-Wirkung der erfindungsgemäßen Verbindungen kann in folgenden Assays gezeigt werden:

**Anti-HCMV- (Anti-Humanes Cytomegalie-Virus) Zytopathogenitätstests**

Die Testverbindungen werden als 50 millimolare (mM) Lösungen in Dimethysulfoxid (DMSO) eingesetzt. Ganciclovir, Foscarnet und Cidofovir dienen als Referenzverbindungen. Nach der Zugabe von jeweils 2 µl der 50, 5, 0.5 und 0.05 mM DMSO-Stammlösungen zu je 98 µl Zellkulturmedium in der Reihe 2 A-H in Doppelbestimmung werden 1:2-Verdünnungen mit je 50 µl Medium bis zur Reihe 11 der 96-Well-Platte durchgerührt. Die Wells in den Reihen 1 und 12 enthalten je 50 µl Medium. In die Wells werden dann je 150 µl einer Suspension von 1 x $10^4$ Zellen (humane Vorhautfibroblasten [NHDF]) pipettiert (Reihe 1 = Zellkontrolle) bzw. in die Reihen 2-12 ein Gemisch von HCMV-infizierten und nichtinfizierten NHDF-Zellen (M.O.I. = 0.001 - 0.002), d.h. 1-2 infiziert Zellen auf 1000 nicht-infizierte Zellen. Die Reihe 12 (ohne Substanz) dient als Viruskontrolle. Die End-Testkonzentrationen liegen bei 250 - 0.0005 µM. Die Platten werden 6 Tage bei 37°C / 5 % $CO_2$ inkubiert, d.h. bis in den Viruskontrollen alle Zellen infiziert sind (100 % cytopathogener Effekt [CPE]). Die Wells werden dann durch Zugabe eines Gemisches von Formalin und Giemsa's Farbstoff fixiert und gefärbt (30 Minuten), mit aqua bidest. gewaschen und im Trockenschrank bei 50°C getrocknet. Danach werden die Platten mit einem Overhead-Mikroskop (Plaque multiplier der Firma Technomara) visuell ausgewertet.

Die folgenden Daten können von den Testplatten ermittelt werden:

$CC_{50}$ (NHDF) = maximale Substanzkonzentration in µM, bei der im Vergleich zur unbehandelten Zellkontrolle keine sichtbaren cytostatischen Effekte auf die Zellen erkennbar sind;

$EC_{50}$ (HCMV) = Substanzkonzentration in µM, die den CPE (cytopathischen Effekt) um 50 % im Vergleich zur unbehandelten Viruskontrolle hemmt;

$$\text{SI (Selektivitätsindex)} = CC_{50}\ (\text{NHDF}) / EC_{50}\ (\text{HCMV}).$$

Repräsentative in-vitro-Wirkdateri für die erfindungsgemäßen Verbindungen sind in Tabelle A wiedergegeben:

**Tabelle A**

| Beispiel-Nr. | NHDF $CC_{50}$ [µM] | HCMV $EC_{50}$ [µM] | SI HCMV |
|---|---|---|---|
| **4** | 21 | 0.0007 | 28767 |
| **9** | 21 | 0.0059 | 3559 |
| **14** | 11 | 0.0016 | 7333 |
| **23** | 11 | 0.0054 | 2637 |

Die Eignung der erfindungsgemäßen Verbindungen zur Behandlung von HCMV-Infektionen kann im folgenden Tiermodell gezeigt werden:

**HCMV Xenograft-Gelfoam®-Modell**

Tiere:

3-4 Wochen alte weibliche immundefiziente Mäuse (16-18 g), Fox Chase SCID oder Fox Chase SCID-NOD oder SCID-beige werden von kommerziellen Züchtern (Bomholtgaard, Jackson) bezogen. Die Tiere werden unter sterilen Bedingungen (einschließlich Streu und Futter) in Isolatoren gehalten.

Virusanzucht:

Humanes Cytomegalievirus (HCMV), Stamm Davis, wird *in vitro* auf humanen embryonalen Vorhautfibroblasten (NHDF-Zellen) angezüchtet. Nach Infektion der NHDF-Zellen mit einer Multiplizität der Infektion (M.O.I) von 0.01 werden die virusinfizierten Zellen 5-7 Tage später geerntet und in Gegenwart von Minimal Essential Medium (MEM), 10 %

foetalem Kälberserum (FKS) mit 10 % DMSO bei -40°C aufbewahrt. Nach serieller Verdünnung der virusinfizierten Zellen in Zehnerschritten erfolgt die Titerbestimmung auf 24-Well-Platten konfluenter NHDF-Zellen nach Vitalfärbung mit Neutralrot oder Fixierung und Färbung mit einem Formalin-Giemsa Gemisch (wie oben beschrieben).

Vorbereitung der Schwämme, Transplantation, Behandlung und Auswertung:

1x1x1 cm große Kollagenschwämme (Gelfoam®; Fa. Peasel & Lorey, Best.-Nr. 407534; K.T. Chong et al., Abstracts of 39th Interscience Conference on Antimicrobial Agents and Chemotherapy, 1999, S. 439; P.M. Kraemer et al., Cancer Research 1983, (43): 4822-4827) werden zunächst mit Phosphat-gepufferter Saline (PBS) benetzt, die eingeschlossenen Luftblasen durch Entgasen entfernt und dann in MEM + 10 % FKS aufbewahrt. 1 x $10^6$ virusinfizierte NHDF-Zellen (Infektion mit HCMV-Davis M.O.I = 0.01) werden 3 Stunden nach Infektion abgelöst und in 20 µl MEM, 10 % FKS auf einen feuchten Schwamm getropft. Optional werden nach 12-13 Stunden auf die infizierten Schwämme 5 ng/µl basic Fibroblast Growth Factor (bFGF) in 25 µl PBS / 0.1% BSA / 1 mM DTT aufgebracht und 1 Stunde inkubiert. Zur Transplantation werden die immundefizienten Mäuse mit Avertin oder einem Gemisch aus Azepromazin-Xylazin und Ketamin narkotisiert, das Rückenfell mit Hilfe eines Trockenrasierers entfernt, die Oberhaut 1-2 cm geöffnet, entlastet und die feuchten Schwämme unter die Rückenhaut transplantiert. Die Operationswunde wird mit Gewebekleber verschlossen. 24 Stunden nach der Transplantation werden die Mäuse über einen Zeitraum von 8 Tagen dreimal täglich (7.00 Uhr und 14.00 Uhr und 19.00 Uhr), zweimal täglich (8.00 Uhr und 17.00 Uhr), oder einmal täglich (14.00 Uhr) peroral mit Substanz behandelt. Die Dosis beträgt 3 oder 10 oder 30 oder 100 mg/kg Körpergewicht, das Applikationsvolumen 10 mL/kg Körpergewicht. Die Formulierung der Substanzen erfolgt in Form einer 0,5 %igen Tylosesuspension optional mit 2 % DMSO. 9 Tage nach Transplantation und 16 Stunden nach der letzten Substanzapplikation werden die Tiere schmerzlos getötet und der Schwamm entnommen. Die virusinfizierten Zellen werden durch Kollagenaseverdau (330 U / 1.5 mL) aus dem Schwamm freigesetzt und in Gegenwart von MEM, 10 % foetalem Kälberserum, 10 % DMSO bei -140°C aufbewahrt. Die Auswertung erfolgt nach serieller Verdünnung der virusinfizierten Zellen in Zehnerschritten durch Titerbestimmung auf 24-Well-Platten konfluenter NHDF-Zellen nach Vitalfärbung mit Neutralrot oder nach Fixierung und Färbung mit einem Formalin-Giemsa Gemisch (wie oben beschrieben). Ermittelt wird die Anzahl infektiöser Viruspartikel nach Substanzbehandlung im Vergleich zur placebobehandelten Kontrollgruppe.

## C. Ausführungsbeispiele für Pharmazeutische Zusammensetzungen

Die erfindungsgemäßen Verbindungen können folgendermaßen in pharmazeutische Zubereitungen überführt werden:

**Tablette:**

Zusammensetzung:

100 mg der Verbindung von Beispiel 1, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.
Tablettengewicht 212 mg. Durchmesser 8 mm, Wölbungsradius 12 mm.

Herstellung:

Die Mischung aus Wirkstoff, Lactose und Stärke wird mit einer 5 %-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben). Als Richtwert für die Verpressung wird eine Presskraft von 15 kN verwendet.

**Oral applizierbare Suspension:**

Zusammensetzung:

1000 mg der Verbindung von Beispiel 1, 1000 mg Ethanol (96 %), 400 mg Rhodigel (Xanthan gum der Fa. FMC, Pennsylvania, USA) und 99 g Wasser.
Einer Einzeldosis von 100 mg der erfindungsgemäßen Verbindung entsprechen 10 mL orale Suspension.

Herstellung:

Das Rhodigel wird in Ethanol suspendiert, der Wirkstoff wird der Suspension zugefügt. Unter Rühren erfolgt die Zugäbe des Wassers. Bis zum Abschluss der Quellung des Rhodigels wird ca. 6 h gerührt.

**Intravenös applizierbare Lösung:**

Zusammensetzung:

1 mg der Verbindung von Beispiel 1, 15 g Polyethylenglykol 400 und 250 g Wasser für Injektionszwecke.

Herstellung:

Die erfindungsgemäße Verbindung wird zusammen mit Polyethylenglykol 400 in dem Wasser unter Rühren gelöst. Die Lösung wird sterilfiltriert (Porendurchmesser 0,22 $\mu$m) und unter aseptischen Bedingungen in hitzesterilisierte Infusionsflaschen abgefüllt. Diese werden mit Infusionsstopfen und Bördelkappen verschlossen.

## Patentansprüche

**1.** Verbindung der Formel

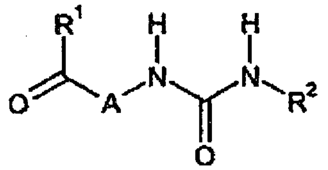

(I),

in welcher

$R^1$ für eine Gruppe der Formel

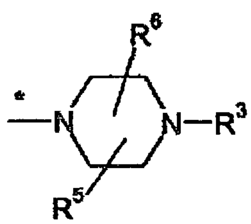

oder

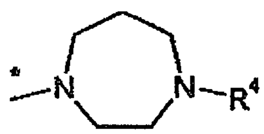

steht,
wobei
* für die Anknüpfstelle an die Carbonyl-Gruppe steht,
$R^3$ für Phenyl oder 5- oder 6-gliedriges Heteroaryl steht,
worin Phenyl und Heteroaryl substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Difluoromethyl, Trifluormethoxy, Difluormethoxy, Monofluormethoxy, Trifluormethylthio, $C_1$-$C_6$-Alkyl, $C_1$-$C_6$-Alkoxy, Hydroxycarbonyl, $C_1$-$C_6$-Alcoxycarbonyl, Amino, $C_1$-$C_6$-Akylamino, Aminocarbonyl und $C_1$-$C_6$-Alkylammocarbonyl,
$R^4$ für Phenyl oder 5- oder 6-gliedriges Heteroaryl steht,
worin Phenyl und Heteroaryl substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Difluormethyl, Trifluormethoxy, Difluormethoxy. Monofluormethoxy, Trifluormethylthio, $C_1$-$C_6$Alkyl, $C_1$-$C_6$-Alkoxy, Hydroxycarbonyl, $C_1$-$C_6$-Alkoxycarbonyl, Amino, $C_1$-$C_6$-Alkylamino, Aminocarbonyl und $C_1$-$C_6$-Alkylaminocarbonyl,
und

$R^5$ und $R^6$ unabhängig voneinander für Wasserstoff, Methyl oder Ethyl stehen,
$R^2$ für Phenyl steht,
wobei Phenyl substituiert sein kann mit 1 bis 3 Substituenten, wobei die Substituenten unabhängig voneinander ausgewählt werden aus der Gruppe, bestehend aus Halogen, Hydroxy, Trifluormethyl, Difluormethyl, Trifluormethoxy, Difluormethoxy, Monofluormethoxy, Trifluormethylthio, $C_1$-$C_6$-Alkyl und $C_1$-$C_6$-Alkoxy,
A für eine Gruppe der Formel

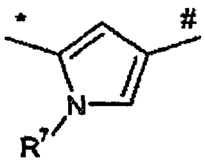 , 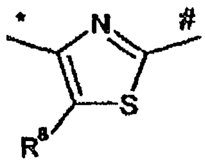 ,

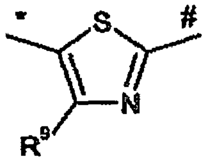 oder 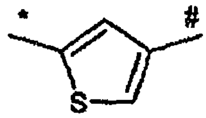

steht,
wobei
* für die Anknupfstelle an die Carbonyl-Gruppe steht,
# für die Anknüpfstelle an das Stickstoffatom des Harnstoffs steht,
$R^7$ für $C_1$-$C_6$-Alkyl steht,
wobei Alkyl substituiert sein kann mit einem Substituenten, wobei der Substituent ausgewählt wird aus der Gruppe, bestehend aus $C_3$-$C_6$-Cycloalkyl, $C_6$-$C_{10}$-Aryl und 5- oder 6-gliedriges Heteroaryl,
worin Cycloalyl, Aryl und Heteroaryl substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Difluormethyl, Trifluormethoxy, Difluormethoxy, Monofluormethoxy, Trifluormethylthio, $C_1$-$C_6$-Alkyl, $C_1$-$C_6$-Alkoxy, Hydroxycarbonyl, $C_1$-$C_6$-Alkoxycarbonyl, Amino, $C_1$-$C_6$-Alkyl-amino, Aminocarbonyl und $C_1$-$C_6$-Alkylaminocarbonyl,
und
$R^8$ und $R^9$ unabhängig voneinander für Wasserstoff, Halogen oder $C_1$-$C_6$-Alkyl stehen,
wobei Alkyl substituiert sein kann mit einem Substituenten, wobei der Substituent ausgewählt wird aus der Gruppe, bestehend aus $C_3$-$C_6$-Cycloalkyl, $C_6$-$C_{10}$-Aryl und 5- oder 6-gliedriges Heteroaryl,
worin Cyctoalkyl, Aryl und Heteroaryl substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl. Diftuormethyl, Trifluormethoxy, Difluormethoxy, Monofluor-methoxy, Trifluormethylthio, $C_1$-$C_6$-Alkyl, $C_1$-$C_6$-Alkoxy, Hydroxycarbonyl, $C_1$-$C_6$-Alkoxycarbonyl, Amino, $C_1$-$C_6$-Alkylamino, Aminocarbonyl und $C_1$-$C_6$-Alkylaminocarbonyl,

oder eines ihrer Salze, ihrer Solvate oder der Solvate ihrer Salze.

2. Verbindung nach Aspruch 1, **dadurch gekennzeichnet, dass**

$R^1$ für eine Gruppe der Formel

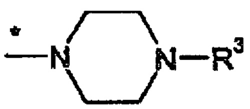

steht,
wobei
* für die Anknüpfstelle an die Carbonyl-Gruppe steht,
$R^3$ für Phenyl oder 5- oder 6-gliedriges Heteroaryl steht,

worin Phenyl und Heteroaryl substituiert sein können mit 1 bis 3 Substituenten, wobei -die Substituenten unabhängig voneinander ausgewählt werden aus der Gruppe, bestehend aus Halogen, Hydroxy, Oxo, Nitro, Cyano, Trifluormethyl, Difluormethyl, Trifluormethoxy, Difluormethoxy, Monofluormethoxy, Trifluormethylthio, $C_1$-$C_6$-Alkyl, $C_1$-$C_6$-Alkoxy, Hydroxycarbonyl, $C_1$-$C_6$-Alkoxycarbonyl, Amino, $C_1$-$C_6$-Alkylamino, Aminocarbonyl und $C_1$-$C_6$-Alkylaminocarbonyl,
$R^2$ für Phenyl steht,
wobei Phenyl substituiert sein kann mit 1 bis 3 Substituenten, wobei die Substituenten unabhängig voneinander ausgewählt werden aus der Gruppe, bestehend aus Halogen, Hydroxy, Trifluormethyl, Difluormethyl, Trifluormethoxy, Difluormethoxy, Monofluormethoxy, Trifluormethylthio, $C_1$-$C_6$-Alkyl und $C_1$-$C_6$-Alkoxy,
A für eine Gruppe der Formel

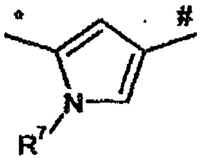 , 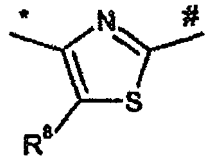 ,

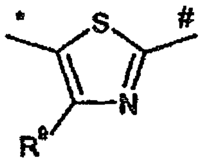 oder 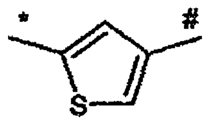

steht, wobei * für die Anknüpfstelle an die Carbonyl-Gruppe steht, # für die Anknüpfstelle an das Stickstoffatom des Harnstoffs steht, $R^7$ für $C_1$-$C_6$-Alkyl steht,
wobei Alkyl substituiert sein kann mit einem Substituenten, wobei der Substituent ausgewählt wird aus der Gruppe, bestehend aus $C_3$-$C_6$-Cycloalkyl, $C_6$-$C_{10}$-Aryl und 5- oder 6-gliedriges Heteroaryl,
worin Cycloalkyl, Aryl und Heteroaryl substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Difluormethyl, Trifluormethoxy, Difluormethoxy, Monofluormethoxy, Trifluormethylthio, $C_1$-$C_6$-Alkyl, $C_1$-$C_6$-Alkoxy, Hydroxycarbonyl, $C_1$-$C_6$-Alkoxycarbonyl, Amino, $C_1$-$C_6$-Alkyl-amino, Aminocarbonyl und $C_1$-$C_6$-Alkylaminocarbonyl,
und
$R^8$ und $R^9$ unabhängig voneinander für Wasserstoff, Halogen oder $C_1$-$C_6$-Alkyl stehen,

oder eines ihrer Salze, ihrer Solvate oder der Solvate ihrer Salze.

3. Verbindung nach Anspruch 1 oder 2, **dadurch gekennzeichnet, dass**

$R^1$ für eine Gruppe der Formel

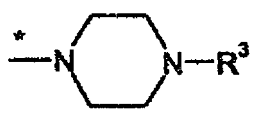

steht,
wobei
* für die Anknüpfstelle an die Carbonyl-Gruppe steht,
$R^3$ für Phenyl oder Pyridyl steht,
worin Phenyl und Pyridyl substituiert sein können mit 1 bis 3 Substituenten, wobei die Substituenten unabhängig voneinander ausgewählt werden aus der Gruppe, bestehend aus Halogen, Nitro, Cyano, Trifluormethyl, Difluormethyl, Trifluomethoxy, Difluormethoxy, Monofluormethoxy, $C_1$-$C_4$-Alkyl und $C_1$-$C_4$-Alkoxy,
$R^2$ für Phenyl steht,
wobei Phenyl substituiert sein kann mit 1 bis 3 Substituenten, wobei die Substituenten unabhängig voneinander ausgewählt werden aus der Gruppe, bestehend aus Fluor, Chlor, Trifluormethoxy, Difluormethoxy, Trifluormethylthio und Methyl,
A für eine Gruppe der Formel

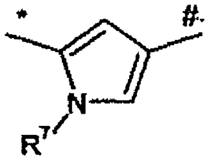
,
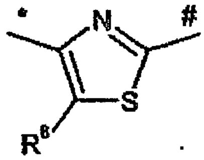
,

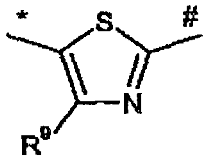
oder
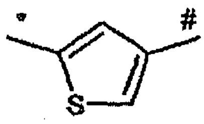

steht,
wobei
* für die Anknüpfstelle an die Carbonyl-Gruppe steht,
# für die Anknüpfstelle an das Stickstoffatom des Harnstoffs steht,
$R^7$ für Methyl, Ethyl oder n-Butyl steht,
wobei Methyl, Ethyl und n-Butyl substituiert sein können mit einem Substituenten, wobei der Substituent ausgewählt wird aus der Gruppe, bestehend aus Cyclopropyl und Phenyl,
worin Phenyl substituiert sein kann mit einem Substituenten Trifluormethyl,
und
$R^8$ und $R^9$ unabhängig voneinander für Wasserstoff, Brom, Chlor, Methyl oder Ethyl stehen,

oder eines ihrer Salze, ihrer Solvate oder der Solvate ihrer Salze.

4. Verfahren zur Herstellung einer Verbindung der Formeln (I) nach Anspruch 1, **dadurch gekennzeichnet, dass** nach Verfahren [A] eine Verbindung der Formel

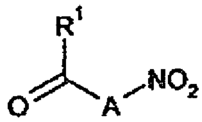
(II),

in welcher

$R^1$ die in Anspruch 1 angegebene Bedeutung hat,

in der ersten Stufe mit einem Reduktionsmittel und in der zweiten Stufe in Gegenwart eines Kohlensäurederivates mit einer Verbindung der Formel

$H_2N\text{-}R^2$ (III),

in welcher

$R^2$ die in Anspruch 1 angegebene Bedeutung hat,

oder
nach Verfahren [B] eine Verbindung der Formel (II) in der ersten Stufe mit einem Reduktionsmittel und in der zweiten Stufe mit einer Verbindung der Formel

$OCN\text{-}R^2$ (IV),

in welcher

$R^2$ die in Anspruch 1 angegebene Bedeutung hat,

oder
nach Verfahren [C] eine Verbindung der Formel

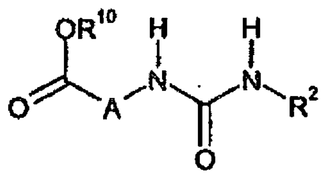 (V),

in welcher

$R^2$ die in Anspruch 1 angegebene Bedeutung hat, und
$R^{10}$ für Methyl oder Ethyl steht,

in der ersten Stufe mit einer Base und in der zweiten Stufe mit einer Verbindung der Formel

$R^1$-H (VI),

in welcher

$R^1$ die in Anspruch 1 angegebene Bedeutung hat,

in Gegenwart von Dehydratisierungsreagenzien umgesetzt wird.

5. Verbindung nach einem der Ansprüche 1 bis 3 zur Behandlung und/oder Prophylaxe von Krankheiten.

6. Arzneimittel enthaltend eine Verbindung nach einem der Ansprüche 1 bis 3 in Kombination mit mindestens einem inerten, nichttoxischen, pharmazeutisch geeigneten Hilfsstoff.

7. Verwendung einer Verbindung nach einem der Ansprüche 1 bis 3 zur Herstellung eines Arzneimittels zur Behandlung und/oder Prophylaxe von Virusinfektionen.

8. Verwendung nach Anspruch 7, **dadurch gekennzeichnet, dass** die Virusinfektion eine Infektion mit dem humanen Cytomegalievirus (HCMV) oder einem anderen Vertreter der Gruppe der Herpes viridae ist.

9. Arzneimittel nach Anspruch 6 zur Behandlung und/oder Prophylaxe von Virusinfektionen.

## Claims

1. A compound having the formula

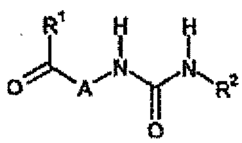 (I),

in which

$R^1$ denotes a group having the formula

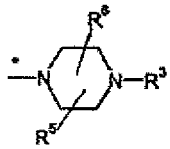

or

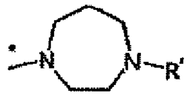

where

* denotes the point of attachment to the carbonyl group

$R^3$ denotes phenyl or 5 or 6-membered heteroaryl wherein phenyl and heteroaryl may be substituted by 1 to 3 substituents, said substituents being chosen independently of each other from the group consisting of halogen, hydroxy, oxo, nitro, cyano, trifluoromethyl, difluoromethyl, trifluoromethoxy, difluoromethoxy, monofluoromethoxy, trifluoromethylthio, $C_1$-$C_6$-alkyl, $C_1$-$C_6$-alkoxy, hydroxycarbonyl, $C_1$-$C_6$-alkoxycarbonyl, amino, $C_1$-$C_6$-alkylamino, aminocarbonyl and $C_1$-$C_6$-alkylaminocarbonyl,

$R^4$ denotes phenyl or 5 or 6-membered heteroaryl,

wherein phenyl and heteroaryl may be substituted by 1 to 3 substituents, said substituents being chosen independently of each other from the group consisting of halogen, hydroxy, oxo, nitro, cyano, trifluoromethyl, difluoromethyl, trifluoromethoxy, difluoromethoxy, monofluoromethoxy, trifluoromethylthio, $C_1$-$C_6$-alkyl, $C_1$-$C_6$-alkoxy, hydroxycarbonyl, $C_1$-$C_6$-alkoxycarbonyl, amino, $C_1$-$C_6$-alkylamino, aminocarbonyl and $C_1$-$C_6$-alkylaminocarbonyl,

and

$R^5$ and $R^6$ denote, independently of each other, hydrogen, methyl or ethyl

$R^2$ denotes phenyl

wherein phenyl may be substituted by 1 to 3 substituents, said substituents being chosen independently of each other from the group consisting of halogen, hydroxy, trifluoromethyl, difluoromethyl, trifluoromethoxy, difluoromethoxy, monofluoromethoxy, trifluoromethylthio, $C_1$-$C_6$-alkyl and $C_1$-$C_6$-alkoxy

A denotes a group having the formula

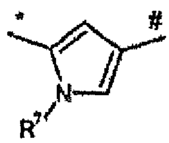

,

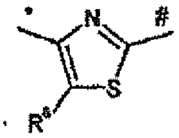

,

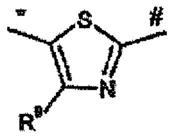

or

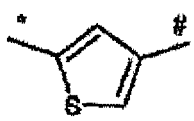

where

* denotes the point of attachment to the carbonyl group

# denotes the point of attachment to the nitrogen atom of the urea

$R^7$ denotes $C_1$-$C_6$-alkyl

wherein alkyl can be substituted by a substituent, said substituent being chosen from the group consisting of $C_3$-$C_6$-cycloalkyl, $C_6$-$C_{10}$-aryl and 5 or 6-membered heteroaryl,

wherein cycloalkyl, aryl and heteroaryl may be substituted by 1 to 3 substituents, said substituents being chosen independently of each other from the group consisting of halogen, hydroxy, oxo, nitro, cyano, trifluoromethyl, difluoromethyl, trifluoromethoxy, difluoromethoxy, monofluoromethoxy, trifluoromethylthio, $C_1$-$C_6$-alkyl, $C_1$-$C_6$-alkoxy, hydroxycarbonyl, $C_1$-$C_6$-alkoxycarbonyl, amino, $C_1$-$C_6$-alkylamino, aminocarbonyl and $C_1$-$C_6$-alkylaminocarbonyl,

and

$R^8$ and $R^9$ denote, independently of each other, hydrogen, halogen or $C_1$-$C_6$-alkyl,

wherein alkyl may be substituted by a substituent, the substituent being chosen from the group consisting of $C_3$-$C_6$-cycloalkyl, $C_6$-$C_{10}$-aryl and 5 or 6-membered heteroaryl,

wherein cycloalkyl, aryl and heteroaryl may be substituted by 1 to 3 substituents, said substituents being chosen

independently of each other from the group consisting of halogen, hydroxy, oxo, nitro, cyano, trifluoromethyl, difluoromethyl, trifluoromethoxy, difluoromethoxy, monofluoromethoxy, trifluoromethylthio, $C_1$-$C_6$-alkyl, $C_1$-$C_6$-alkoxy, hydroxycarbonyl, $C_1$-$C_6$-alkoxycarbonyl, amino, $C_1$-$C_6$-alkylamino, aminocarbonyl and $C_1$-$C_6$-alkylaminocarbonyl,

or one of its salts, its solvates, or solvates of its salts.

**2.** A compound according to Claim 1, **characterized in that**

$R^1$ denotes a group having the formula

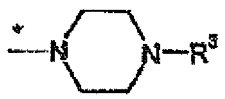

where
* denotes the point of attachment to the carbonyl group
$R^3$ denotes phenyl or 5 or 6-membered heteroaryl
wherein phenyl and heteroaryl may be substituted by 1 to 3 substituents, said substituents being chosen independently of each other from the group consisting of halogen, hydroxy, oxo, nitro, cyano, trifluoromethyl, difluoromethyl, trifluoromethoxy, difluoromethoxy, monofluoromethoxy, trifluoromethylthio, $C_1$-$C_6$-alkyl, $C_1$-$C_6$-alkoxy, hydroxycarbonyl, $C_1$-$C_6$-alkoxycarbonyl, amino, $C_1$-$C_6$-alkylamino, aminocarbonyl and $C_1$-$C_6$-alkylaminocarbonyl
$R^2$ denotes phenyl
wherein phenyl and heteroaryl may be substituted by 1 to 3 substituents, said substituents being chosen independently of each other from the group consisting of halogen, hydroxy, trifluoromethyl, difluoromethyl, trifluoromethoxy, difluoromethoxy, monofluoromethoxy, trifluoromethylthio, $C_1$-$C_6$-alkyl and $C_1$-$C_6$-alkoxy,
A denotes a group having the formula

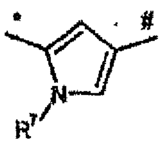
,
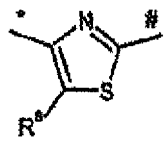
,
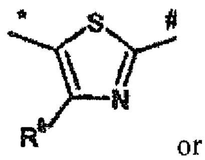
or
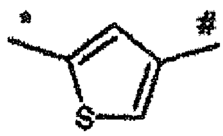

where
* denotes the point of attachment to the carbonyl group
\# denotes the point of attachment to the nitrogen atom of the urea
$R^7$ denotes $C_1$-$C_6$-alkyl
wherein alkyl may be substituted by a substituent, said substituent being chosen from the group consisting of $C_3$-$C_6$-cycloalkyl, $C_6$-$C_{10}$-aryl and 5 or 6-membered heteroaryl,
wherein cycloalkyl, aryl and heteroaryl may be substituted by 1 to 3 substituents, said substituents being chosen independently of each other from the group consisting of halogen, hydroxy, oxo, nitro, cyano, trifluoromethyl, difluoromethyl, trifluoromethoxy, difluoromethoxy, monofluoromethoxy, trifluoromethylthio, $C_1$-$C_6$-alkyl, $C_1$-$C_6$-alkoxy, hydroxycarbonyl, $C_1$-$C_6$-alkoxycarbonyl, amino, $C_1$-$C_6$-alkylamino, aminocarbonyl and $C_1$-$C_6$-alkylaminocarbonyl,
and
$R^8$ and $R^9$ denote, independently of each other, hydrogen, halogen or $C_1$-$C_6$-alkyl

or one of its salts, its solvates, or the solvates of its salts.

3. A compound according to Claim 1 or 2, **characterized in that**

$R^1$ denotes a group having the formula

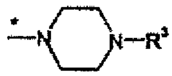

where
* denotes the point of attachment to the carbonyl group
$R^3$ denotes phenyl or pyridyl
wherein phenyl and pyridyl may be substituted by 1 to 3 substituents, said substituents being chosen independently of each other from the group consisting of halogen, nitro, cyano, trifluoromethyl, difluoromethyl, trifluoromethoxy, difluoromethoxy, monofluoromethoxy, $C_1$-$C_4$-alkyl and $C_1$-$C_4$-alkoxy,
$R^2$ denotes phenyl
wherein phenyl may be substituted by 1 to 3 substituents, said substituents being chosen independently of each other from the group consisting of fluorine, chlorine, trifluoromethoxy, difluoromethoxy, trifluoromethylthio and methyl,
A denotes a group having the formula

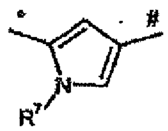 , 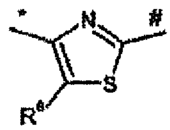 ,

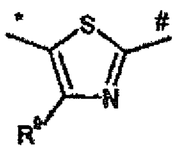 or 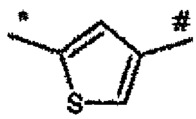

where
* denotes the point of attachment to the carbonyl group
# denotes the point of attachment to the nitrogen atom of the urea
$R^7$ denotes methyl, ethyl or n-butyl
wherein methyl, ethyl and n-butyl may be substituted by a substituent, said substituent being chosen from the group consisting of cyclopropyl and phenyl
wherein phenyl may be substituted by a substituent, trifluoromethyl,
and
$R^8$ and $R^9$ denote, independently of each other, hydrogen, bromine, chlorine, methyl or ethyl

or one of its salts, its solvates, or the solvates of its salts.

4. A method for producing a compound having the formula (I) according to Claim 1, **characterized in that** in accordance with method [A] a compound having the formula

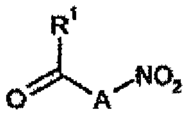 (II),

in which

$R^1$ has the same meaning as that given in Claim 1,

is reacted in the first step with a reducing agent and in the second step is reacted in the presence of a carbonic acid derivative with a compound having the formula

$H_2N-R^2$ (III)

in which

$R^2$ has the same meaning as that given in Claim 1

or
in accordance with method [B] a compound having the formula (II) is reacted in the first stage with a reducing agent and in the second stage is reacted with a compound having the formula

$OCN-R^2$ (IV),

in which

$R^2$ has the same meaning as that given in Claim 1,

or
in accordance with method [C] a compound having the formula

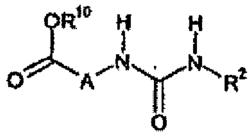 (V),

in which

$R^2$ has the same meaning as that given in Claim 1, and
$R^{10}$ denotes methyl or ethyl

is reacted in the first stage with a base and in the second stage with a compound having the formula

$R^1-H$ (VI)

in which

$R^1$ has the same meaning as that given in Claim 1,

said reactions occurring in the presence of dehydrating reagents.

**5.** A compound according to any of Claims 1 to 3 for the treatment and/or prophylaxis of diseases.

**6.** A medication containing a compound according to any of the Claims 1 to 3 in combination with at least one inert, non-toxic, pharmaceutically suitable excipient.

**7.** The use of a compound according to any of Claims 1 to 3 for producing a medication for the treatment and/or prophylaxis of viral infections.

**8.** The use according to Claim 7, **characterized in that** the viral infection is an infection with the human cytomegalovirus (HCMV) or another representative of the *Herpes viridae* group.

**9.** A medication according to Claim 6 for the treatment and/or prophylaxis of viral infections.

## Revendications

**1.** Un composé de la formule

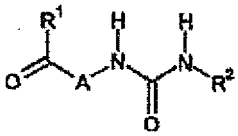 (I),

dans lequel

$R^1$ désigne un groupe de la formule

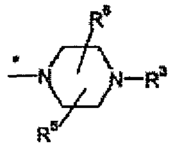 or 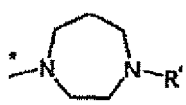

dans lequel
* désigne le point d'attachement au groupe carbonyle,
$R^3$ désigne phényle ou hétéroaryle à 5 ou 6 chaînons,
dans lequel phényle et hétéroaryle peuvent être substitués par 1 à 3 substituants, lesdits substituants étant choisis indépendamment les uns des autres parmi le groupe composé d'halogène, hydroxy, oxo, nitro, cyano, trifluorométhyle, difluorométhyle, trifluorométhoxy, difluorométhoxy, monofluorométhoxy, trifluorométhylthio, $C_1$-$C_6$-alkyle, $C_1$-$C_6$-alkoxy, hydroxycarbonyle, $C_1$-$C_6$-alkoxycarbonyle, amino, $C_1$-$C_6$-alkylamino, aminocarbonyle et $C_1$-$C_6$-alkylaminocarbonyle,
$R^4$ désigne phényle ou hétéroaryle à 5 ou 6 chaînons,
dans lequel phényle et hétéroaryle peuvent être substitués par 1 à 3 substituants, lesdits substituants étant choisis indépendamment les uns des autres parmi le groupe composé d'halogène, hydroxy, oxo, nitro, cyano, trifluorométhyle, difluorométhyle, trifluorométhoxy, difluorométhoxy, monofluorométhoxy, trifluorométhylthio, $C_1$-$C_6$-alkyle, $C_1$-$C_6$-alkoxy, hydroxycarbonyle, $C_1$-$C_6$-alkoxycarbonyle, amino, $C_1$-$C_6$-alkylamino, aminocarbonyle et $C_1$-$C_6$-alkylaminocarbonyle,
et
$R^5$ et $R^6$ désignent, indépendamment l'un de l'autre, hydrogène, méthyle ou éthyle,
$R^2$ désigne phényle,
dans lequel phényle peut être substitué par 1 à 3 substituants, lesdits substituants étant choisis indépendamment les uns des autres parmi le groupe composé d'halogène, hydroxy, trifluorométhyle, difluorométhyle, trifluorométhoxy, difluorométhoxy, monofluorométhoxy, trifluorométhylthio, $C_1$-$C_6$-alkyle, $C_1$-$C_6$-alkoxy
A désigne un groupe de la formule

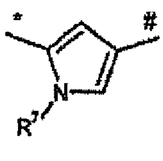 , 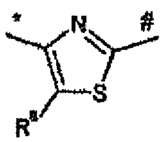 ,

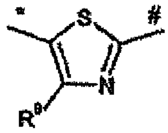 or 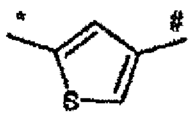

dans lequel
* désigne le point d'attachement au groupe carbonyle,
# désigne le point d'attachement l'atome de nitrogène de l'urée,

$R^7$ désigne $C_1$-$C_6$-alkyle,
dans lequel alkyle peut être substitué par 1 à 3 substituants, ledit substituant étant choisi parmi le groupe composé de $C_3$-$C_6$-cycloalkyle, $C_6$-$C_{10}$-aryle et hétéroaryle de 5 à 6 chaînons,
dans lequel cycloalkyle, aryle et hétéroaryle peuvent être substitués par 1 à 3 substituants, lesdits substituants étant choisis indépendamment les uns des autres parmi le groupe composé d'halogène, hydroxy, oxo, nitro, cyano, trifluorométhyle, difluorométhyle, trifluorométhoxy, difluorométhoxy, monofluorométhoxy, trifluorométhylthio, $C_1$-$C_6$-alkoxy, hydroxycarbonyle, $C_1$-$C_6$-alkoxycarbonyle, amino, $C_1$-$C_6$-alkylamino, aminocarbonyl et $C_1$-$C_6$-alkylaminocarbonyle,
et
$R^8$ et $R^9$ désignent, indépendamment l'un de l'autre, hydrogène, halogène ou $C_1$-$C_6$-alkyle,
dans lequel alkyle peut être substitué par un substituant, ledit substituant étant choisi parmi le groupe composé de $C_3$-$C_6$-cycloalkyle, $C_6$-$C_{10}$-aryle et hétéroaryle de 5 à 6 chaînons,
dans lequel cycloalkyle, aryle et hétéroaryle peuvent être substitués par 1 à 3 substituants, lesdits substituants étant choisis indépendamment les uns des autres parmi le groupe composé d'halogène, hydroxy, oxo, nitro, cyano, trifluorométhyle, difluorométhyle, trifluorométhoxy, difluorométhoxy, monofluorométhoxy, trifluorométhylthio, $C_1$-$C_6$-alkoxy, hydroxycarbonyle, $C_1$-$C_6$-alkoxycarbonyle, amino, $C_1$-$C_6$-alkylamino, aminocarbonyle et $C_1$-$C_{6-}$alkylaminocarbonyle,

ou l'un de ses sels, solvates, ou solvates de ses sels.

2. Un composé selon la revendication 1, **caractérisé en ce que**

$R^1$ désigne un groupe de la formule

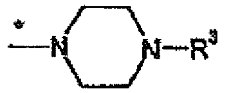

dans lequel
* désigne le point d'attachement au groupe carbonyle
$R^3$ désigne phényle ou hétéroaryle à 5 ou 6 chaînons
dans lequel phényle et hétéroaryle peuvent être substitués par 1 à 3 substituants, lesdits substituants étant choisis indépendamment les uns des autres parmi le groupe composé d'halogène, hydroxy, oxo, nitro, cyano, trifluorométhyle, difluorométhyle, trifluorométhoxy, difluorométhoxy, monofluorométhoxy, trifluorométhylthio, $C_1$-$C_6$-alkyle, $C_1$-$C_6$-alkoxy, hydroxycarbonyle, $C_1$-$C_6$-alkoxycarbonyle, amino, $C_1$-$C_{6-}$alkylamino, aminocarbonyle et $C_1$-$C_{6-}$alkylaminocarbonyle
$R^2$ désigne phényle
dans lequel phényle et hétéroaryle peuvent être substitués par 1 à 3 substituants, lesdits substituants étant choisis indépendamment les uns des autres parmi le groupe composé d'halogène, hydroxy, trifluorométhyle, difluorométhyle, trifluorométhoxy, difluorométhoxy, monofluorométhoxy, trifluorométhylthio, $C_1$-$C_6$-alkyle et $C_1$-$C_6$-alkoxy,
A désigne un groupe de la formule

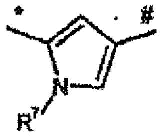 , 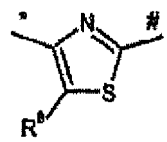 ,

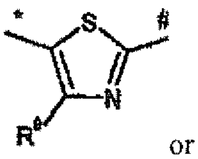 or 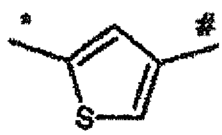

dans lequel

* désigne le point d'attachement au groupe carbonyle
\# désigne le point d'attachement à l'atome de nitrogène de l'urée
$R^7$ désigne $C_1$-$C_6$-alkyle
dans lequel alkyle peut être substitué par un substituant, ledit substituant étant choisi parmi le groupe composé de $C_3$-$C_6$-cycloalkyle, $C_6$-$C_{10}$-aryle et hétéroaryle à 5 ou 6 chaînons,
dans lequel cycloalkyle, aryle et hétéroaryle peuvent être substitués par 1 à 3 substituants, lesdits substituants étant choisis indépendamment les uns des autres parmi le groupe composé d'halogène, hydroxy, oxo, nitro, cyano, trifluorométhyle, difluorométhyle, trifluorométhoxy, difluorométhoxy, monofluorométhoxy, trifluorométhylthio, C1-C6-alkoxy, hydroxycarbonyle, $C_1$-$C_6$-alkoxycarbonyle, amino, $C_1$-$C_6$-alkylamino, aminocarbonyl et $C_1$-$C_6$-alkylaminocarbonyle,
et
$R^8$ et $R^9$ désignent, indépendamment les uns des autres, hydrogène, halogène ou $C_1$-$C_6$-alkyle,

ou l'un de ses sels, solvates, ou solvates de ses sels.

3. Un composé selon la revendication 1 ou 2, **caractérisé en ce que**

$R^1$ désigne un groupe de la formule

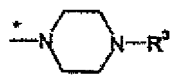

dans lequel
* désigne le point d'attachement au groupe carbonyle
$R^3$ désigne phényle ou pyridyle
dans lequel phényle et pyridyle peuvent être substitués par 1 à 3 substituants, lesdits substituants étant choisis indépendamment les uns des autres parmi le groupe consistant d'halogène, nitro, cyano, trifluorométhyle, difluorométhyle, trifluorométhoxy, difluorométhoxy, monofluorométhoxy, $C_1$-$C_6$-alkyle et $C_1$-$C_4$-alkoxy,
$R^2$ désigne phényle
dans lequel phényle peut être substitué par 1 à 3 substituants, lesdits substituants étant choisis indépendamment les uns des autres parmi le groupe composé de fluor, chlore, trifluorométhoxy, difluorométhoxy, trifluorométhylthio et méthyle,
A désigne un groupe de la formule

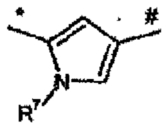 , 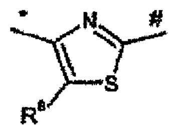 ,

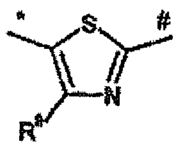 ou 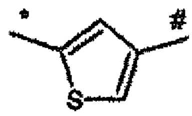

dans lequel
* désigne le point d'attachement au groupe carbonyle,
\# désigne le point d'attachement à l'atome de nitrogène de l'urée,
$R^7$ désigne méthyle, éthyle ou n-butyle,
dans lequel méthyle, éthyle ou n-butyle peut être substitué par un substituant, ledit substituant étant choisi parmi le groupe composé de cyclopropyle et phényle,
dans lequel phényle peut être substitué par un substituant, trifluorométhyle,
et
$R^8$ et $R^9$ désignent, indépendamment l'un de l'autre, hydrogène, brome, chlore, méthyle ou éthyle,

ou l'un de ses sels, solvates, ou solvates de ses sels.

**4.** Un procédé pour produire un composé de la formule (I) selon la revendication 1, **caractérisé en ce que** selon le procédé [A], on fait réagir, dans une première étape, un composé de la formule

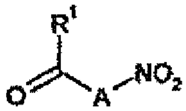 (II),

dans lequel

$R^1$ a le même sens que celui donné dans la revendication 1,

avec un agent de réduction et, dans une seconde étape, en présence d'un dérivé d'acide carbonique, avec un composé de la formule

$H_2N\text{-}R^2$ (III)

dans lequel

$R^2$ a le même sens que celui donné dans la revendication 1,

ou
selon le procédé [B], on fait réagir, dans une première étape, un composé de la formule (II) avec un agent de réduction et, dans une seconde étape, avec un composé de la formule

$OCN\text{-}R^2$ (IV),

dans lequel

$R^2$ a le même sens que celui donné dans la revendication 1,

ou
selon le procédé [C], on fait réagir, dans une première étape, un composé de la formule

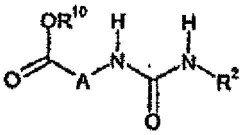 (V),

dans lequel

$R^2$ a le même sens que celui donné dans la revendication 1, et
$R^{10}$ désigne méthyl ou éthyle

avec une base et, dans une seconde étape, avec un composé de la formule

$R^1\text{-}H$ (VI)

dans lequel

$R^1$ a le même sens que celui donné dans la revendication 1,

lesdites réactions se produisant en présence de réactifs de déshydratation.

**5.** Un composé selon une quelconque des revendications 1 à 3 pour le traitement et/ou la prévention de maladies.

**6.** Un médicament contenant un composé selon une quelconque des revendications 1 à 3 en combinaison avec au

moins un excipient inerte, non toxique, pharmaceutiquement approprié.

**7.** L'utilisation d'un composé selon une quelconque des revendications 1 à 3 pour la production d'un médicament pour le traitement et/ou la prévention d'infections virales.

**8.** L'utilisation selon la revendication 7, **caractérisée en ce que** l'infection virale est une infection avec le cytomégalovirus humain (HCMV) ou un autre représentant du groupe *Herpes viridae.*

**9.** Un médicament selon la revendication 6 pour le traitement et/ou la prévention d'infections virales.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9923091 A
- WO 04052852 A


### In der Beschreibung aufgeführte Nicht-Patentliteratur


- **J. CINATL et al.** *FEMS Microbiology Reviews,* 2004, vol. 28, 59-77